# EUROPEAN PATENT SPECIFICATION

(11) **EP 4 446 434 B1**
(45) Date of publication and mention of the grant of the patent: **22.07.2026**
(21) Application number: 22904129.8
(22) Date of filing: 01.12.2022
(51) Int. Cl.: C12Q 1/6858, C12Q 1/686, C12N 15/11, C08G 75/22, C08L 39/00, C08L 81/06, C09D 181/06, C09J 181/06, C12Q 1/6827

(54) **METHOD FOR DETECTING MUTATION IN TARGET BASE SEQUENCE OF NUCLEIC ACID, METHOD FOR SELECTIVELY INHIBITING AMPLIFICATION OF NUCLEIC ACID, AND KITS FOR PERFORMING SAME**
VERFAHREN ZUM NACHWEIS VON MUTATIONEN IN DER ZIELBASENSEQUENZ VON NUKLEINSÄUREN, VERFAHREN ZUR SELEKTIVEN HEMMUNG DER AMPLIFIKATION VON NUKLEINSÄUREN UND KITS ZUR DURCHFÜHRUNG DAVON
PROCÉDÉ DE DÉTECTION D'UNE MUTATION DANS UNE SÉQUENCE DE BASES CIBLE D'ACIDE NUCLÉIQUE, PROCÉDÉ D'INHIBITION SÉLECTIVE DE L'AMPLIFICATION D'ACIDE NUCLÉIQUE, ET KITS POUR LA MISE EN OEUVRE DE CELUI-CI

(30) Priority: 10.12.2021 JP 2021201175
(43) Date of publication of application: 16.10.2024
(73) Proprietor: Nitto Boseki Co., Ltd., Fukushima-shi, Fukushima 960-8161 (JP)
(72) Inventor: FUJIMURA Nahohiro, Kawasaki-shi, Kanagawa 210-0821 (JP); TACHIBANA Ami, Kawasaki-shi, Kanagawa 210-0821 (JP); UCHIKI Tomoaki, Kawasaki-shi, Kanagawa 210-0821 (JP); TAKEUCHI Minoru, Koriyama-shi, Fukushima 963-8061 (JP); TERUUCHI Yoko, Koriyama-shi, Fukushima 963-8061 (JP); WATANABE Koji, Koriyama-shi, Fukushima 963-8061 (JP)
(74) Representative: Hoffmann Eitle
(86) International application number: PCT/JP2022/044359
(87) International publication number: WO 2023/106198

(56) References cited:
- EP-A1- 2 578 618
- WO-A1-03/018841
- WO-A1-2012/033190
- WO-A1-2012/033190
- WO-A1-2014/051076
- WO-A1-2014/051076
- WO-A1-2016/167320
- JP-A- 2005 060 491
- JP-A- 2005 060 491
- JP-A- 2007 204 599
- JP-A- 2008 190 091
- TACHIBANA AMI ET AL: "Cationic copolymers that enhance wild-type-specific suppression in BNA-clamp PCR and preferentially increase the T m of fully matched complementary DNA and BNA strands", vol. 7, no. 1, 10 January 2022 (2022-01-10), XP093068867, Retrieved from the Internet <URL:https://academic.oup.com/biomethods/article-pdf/7/1/bpac009/43858911/bpac009.pdf> DOI: 10.1093/biomethods/bpac009
- TACHIBANA AMI, FUJIMURA NAHOHIRO, TAKEUCHI MINORU, WATANABE KOJI, TERUUCHI YOKO, UCHIKI TOMOAKI: "Cationic copolymers that enhance wild-type-specific suppression in BNA-clamp PCR and preferentially increase the T m of fully matched complementary DNA and BNA strands", BIOLOGY METHODS AND PROTOCOLS, vol. 7, no. 1, 10 January 2022 (2022-01-10), XP093068867, DOI: 10.1093/biomethods/bpac009

## Description

### TECHNICAL FIELD

The invention is set out in the appended set of claims.

### BACKGROUND ART

Mutations in genes may cause cancer, and early discovery of such mutations in genes is expected to lead to early treatment of cancer. A certain genetic mutation has been also found to be greatly involved in susceptibility to certain kinds of diseases, therapeutic effects of drugs, strength of side effects, and the like. For example, Gefitinib (trade name: Iressa), which is a tyrosine kinase inhibitor of epidermal growth factor receptor (EGFR), is known to show great efficacy in some lung cancer patients having a specific genetic mutation, while causing severe interstitial pneumonia in about 0.5% of patients (Non-Patent Literature 1). In colorectal cancer, it is known that when there are mutations in the KRAS gene, the response rate of cetuximab (trade name: Erbitux), a molecularly targeted drug, is low, whereas the response rate is high when there is no mutation in that gene. Therefore, if the efficacy and side effects can be predicted for each patient, it is possible to perform effective treatment with fewer side effects while refraining from administration to a patient in whom efficacy cannot be expected, or a patient in whom serious side effects are expected.

From this perspective, it is recognized that clarifying gene mutations is important in terms of early discovery of cancer, improvement of treatment efficiency, and the like, and various technologies for detecting genetic mutation have been developed in order to meet such needs. Particularly, remarkable progress has been seen in gene amplification technologies such as PCR methods and comprehensive gene analysis technologies such as NGS.

However, genes extracted from a tumor specimen contains a large amount of wild-type gene and a trace amount of mutant gene, but many conventional methods for detecting mutant genes still do not have sufficient sensitivity for detecting a trace amount of mutant gene. For example, as one of methods for detecting gene mutations, there is a method comprising amplifying a mutant gene and a wild-type gene without separating them, and then separating the mutant gene from the wild-type gene by an electrophoresis method, a hybridization method, or the like. However, this method has difficulty of detecting an extremely small amount of mutant gene in the wild-type genes with sufficient sensitivity and accuracy.

On the other hand, a so-called "clamp method" has been developed in which mutant genes are selectively amplified in the stage of gene amplification by inhibiting amplification of a wild-type gene using an artificial oligonucleotide having a base sequence complementary to a standard sequence of the wild-type gene. The artificial nucleic acid used in this method is referred to as a clamp nucleic acid, and has characteristics that the artificial nucleic acid (i) strongly hybridizes with a wild-type gene, but (ii) does not strongly hybridize with mutant genes, and (iii) is less likely to be degraded in a nucleic acid amplification process. When real-time PCR is performed using this nucleic acid, that mutations are considered detectable until the frequency of mutant genes reaches 1%, and when a direct sequencing method is performed using the amplified product of real-time PCR as a template, and mutations are considered detectable until the frequency of mutant genes reaches 0.1% (for example, Patent Literature 1, Non-Patent Literature 2, and Non-Patent Literature 3). As clamp nucleic acids, a peptide nucleic acid (PNA), a locked nucleic acid (LNA), and a bridged nucleic acid (BNA) have been developed, and amplification methods such as a PNA-LNA clamp PCR method and a BNA clamp PCR method have been developed (see, for example, Non-Patent Literatures 2, 3, and 6 and Patent Literatures 1 to 9.).

However, even with these clamp methods, when real-time PCR is performed in conditions where the ratio of mutant genes is very lower compared to the ratio of wild-type genes in a sample, the wild-type genes may be amplified, and the wild-type genes and the mutant genes may not be distinguished from each other. In this regard, when a direct sequencing method is performed with the amplified product of clamp PCR as a template, detection sensitivity of the mutant genes can be increased, but that sequencing is unsuitable for clinical fields where it is required to immediately process a large number of specimens.

Patent Literature 12 relates to a method of detecting a difference in the base sequences by using a clamp nucleic acid in nucleic acid amplification techniques, wherein the clamp nucleic acid is an oligonucleotide analog containing one or more of one or more kinds of unit structures of nucleoside analogs represented by formula (I), formula (II), formula (III) or formula (VI).

### CITATION LIST

### PATENT LITERATURE

PATENT LITERATURE 1: JP-B-6242336
PATENT LITERATURE 2: JP-B-5813263
PATENT LITERATURE 3: JP-B-4731324
PATENT LITERATURE 4: JP-B-4383178
PATENT LITERATURE 5: JP-B-5030998
PATENT LITERATURE 6: JP-B-4151751
PATENT LITERATURE 7: JP-A-2001-89496
PATENT LITERATURE 8: WO 2003/068795
PATENT LITERATURE 9: WO 2005/021570
PATENT LITERATURE 10: JP-B-3756313
PATENT LITERATURE 11: WO 2016/167320
PATENT LITERATURE 12: WO 2014/051076

### NON-PATENT LITERATURE

NON-PATENT LITERATURE 1: Thomas J., et.al., 2004, The NEW ENGLAND JURNAL of MEDICINE, 350; 21
NON-PATENT LITERATURE 2: Nagai K., et.al., 2005, Cancer Research, 65:7276-7282
NON-PATENT LITERATURE 3: Nishino K. et al., 2019, Guide for EGFR gene mutation testing in lung cancer patients, Edition 4.1
NON-PATENT LITERATURE 4: Luming Z. et al., 2011 BioTechniques, 50:311-318
NON-PATENT LITERATURE 5: Nagakubo Y. et al., 2019, BMC Medical Genomics, 12:162
NON-PATENT LITERATURE 6: Murina F.F. et al., 2020, Molecules, 25(4):786

### SUMMARY OF INVENTION

### TECHNICAL PROBLEM

As described above, there is a need for a method capable of detecting mutant genes even in conditions where the ratio of mutant genes is very lower compared to the ratio of wild-type genes in a sample in clinical or research sites, and research is in progress to address this need (Patent Literature 2, Non-Patent Literature 4, and Non-Patent Literature 5). However, the conventional methods require expensive equipment and two or more complicated analysis steps to detect mutant genes with high sensitivity (Non-Patent Literature 2), and there is therefore a demand for a method capable of detecting mutant genes in a sample with high sensitivity by a simple process in clinical and research sites.

An object of the present disclosure is thus to provide a simple method capable of detecting mutant genes at a mutant gene content lower than the detection limit of mutant genes by the conventional clamping methods; and a kit for implementing the method.

It is another object of the present disclosure to provide a simple method capable of selectively inhibiting amplification of a target base sequence in a nucleic acid amplification reaction; and a kit for implementing the method.

### SOLUTION TO PROBLEM

While repeatedly conducting studies for increasing sensitivity for the detection of mutant genes by a clamping method, the present inventors conducted a nucleic acid amplification reaction in the presence of a specific diallylamine/sulfur dioxide copolymer together with a clamp nucleic acid. As a result, the effect of the clamp nucleic acid that selectively inhibits the amplification of a wild-type gene was enhanced by the presence of the diallylamine/sulfur dioxide copolymer, and thereby the present inventors succeeded in increasing sensitivity for the detection of mutant genes.

That is, the present invention provides
a method for detecting mutation in a target base sequence of a nucleic acid in a sample relative to a standard base sequence, comprising the steps of:
subjecting the nucleic acid in the sample to a nucleic acid amplification reaction as a template with
a clamp nucleic acid having a base sequence complementary to the standard base sequence and containing at least one artificial nucleotide residue, and
a diallylamine/sulfur dioxide copolymer that comprises
a constituent unit (A) having a structure represented by general formula (I-a) or general formula (I-b), or a structure of an acid addition salt thereof: 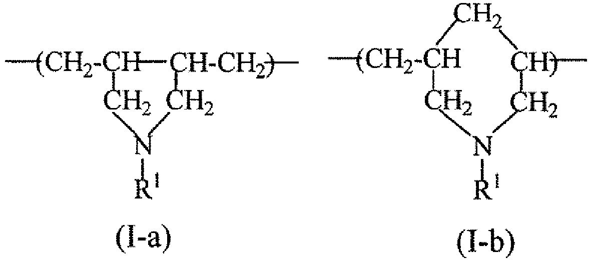
wherein R¹ represents an alkyl group having 1 to 10 carbon atoms, and
a constituent unit (B) having a structure represented by general formula (I-c): 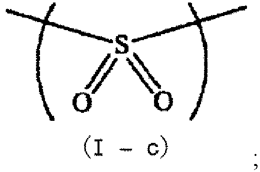 and
determining the presence of the mutation based on the total amount of nucleic acid amplification products obtained by the nucleic acid amplification reaction, the number of amplification cycles of the nucleic acid amplification reaction required for the total amount of the nucleic acid amplification products to reach a threshold value, the amount of nucleic acids with the mutation in the nucleic acid amplification products, or the number of amplification cycles of the nucleic acid amplification reaction required for the amount of nucleic acids with the mutations in the nucleic acid amplification products to reach a threshold value.

The present invention also provides a kit for detecting mutation in a target base sequence of a nucleic acid in a sample relative to a standard base sequence, comprising:
a clamp nucleic acid having a base sequence complementary to the standard base sequence and containing at least one artificial nucleotide residue; and
a diallylamine/sulfur dioxide copolymer that comprises
a constituent unit (A) having a structure represented by general formula (I-a) or general formula (I-b), or a structure of an acid addition salt thereof: 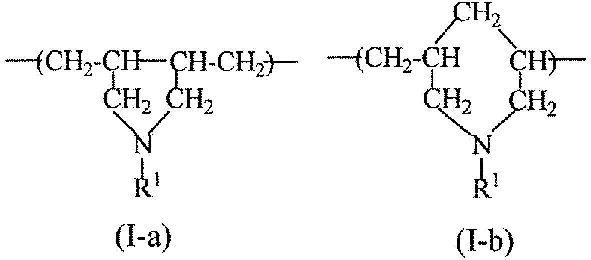
wherein R¹ represents an alkyl group having 1 to 10 carbon atoms, and
a constituent unit (B) having a structure represented by general formula (I-c):

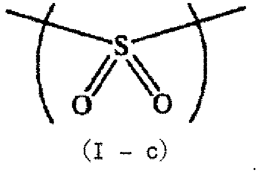

The present invention also provides a method for inhibiting amplification of a nucleic acid in a sample with a predetermined target base sequence in a nucleic acid amplification reaction, the method comprising
subjecting the nucleic acid to a nucleic acid amplification reaction as a template with
a clamp nucleic acid having a base sequence complementary to the target base sequence and containing at least one artificial nucleotide residue, and
a diallylamine/sulfur dioxide copolymer that comprises
a constituent unit (A) having a structure represented by general formula (I-a) or general formula (I-b), or a structure of an acid addition salt thereof: 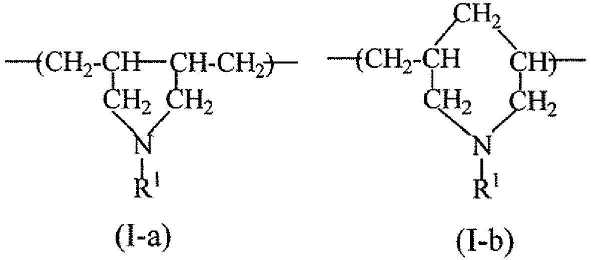
wherein R¹ represents an alkyl group having 1 to 10 carbon atoms, and
a constituent unit (B) having a structure represented by general formula (I-c):

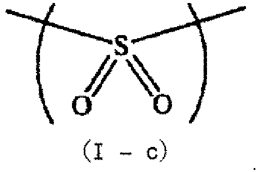

The present invention also provides a kit for inhibiting amplification of a nucleic acid having a target base sequence in a nucleic acid amplification reaction, comprising:
a clamp nucleic acid having a base sequence complementary to the target base sequence and containing at least one artificial nucleotide residue; and
a diallylamine/sulfur dioxide copolymer that comprises
a constituent unit (A) having a structure represented by general formula (I-a) or general formula (I-b), or a structure of an acid addition salt thereof: 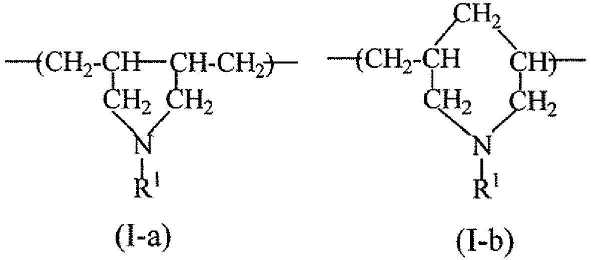
wherein R¹ represents an alkyl group having 1 to 10 carbon atoms, and
a constituent unit (B) having a structure represented by general formula (I-c):

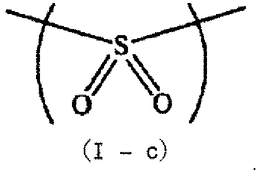

The present invention also provides the use of a nucleic acid amplification inhibition enhancing agent for enhancing a nucleic acid amplification inhibitory effect by a clamp nucleic acid, comprising:
a diallylamine/sulfur dioxide copolymer that comprises
a constituent unit (A) having a structure represented by general formula (I-a) or general formula (I-b), or a structure of an acid addition salt thereof: 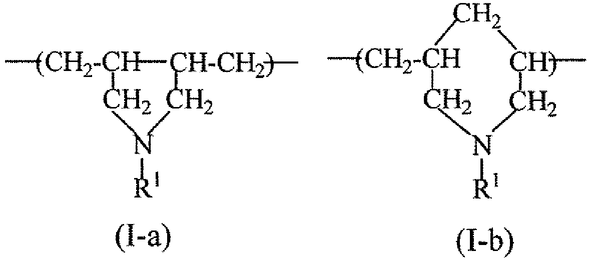
wherein R¹ represents an alkyl group having 1 to 10 carbon atoms, and
a constituent unit (B) having a structure represented by general formula (I-c):

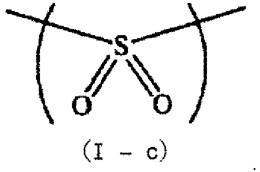

The diallylamine/sulfur dioxide copolymer itself does not inhibit a nucleic acid amplification reaction, but can selectively enhance the effect of inhibiting amplification of a nucleic acid having a sequence complementary to a clamp nucleic acid by the clamp nucleic acid. Therefore, according to one embodiment of the present disclosure, even when the ratio of a nucleic acid having mutations relative to a standard base sequence complementary to a clamp nucleic acid in a sample is very lower in relate to that of a nucleic acid having the standard base sequence in the sample (for example, 0.1% or less), amplification of the nucleic acid having the standard base sequence is selectively inhibited by the clamp nucleic acid, while the nucleic acid having mutations relative to the standard base sequence is amplified as usual, so that a nucleic acid having the mutations can be detected with very high sensitivity by a simple method.

### BRIEF DESCRIPTION OF DRAWINGS

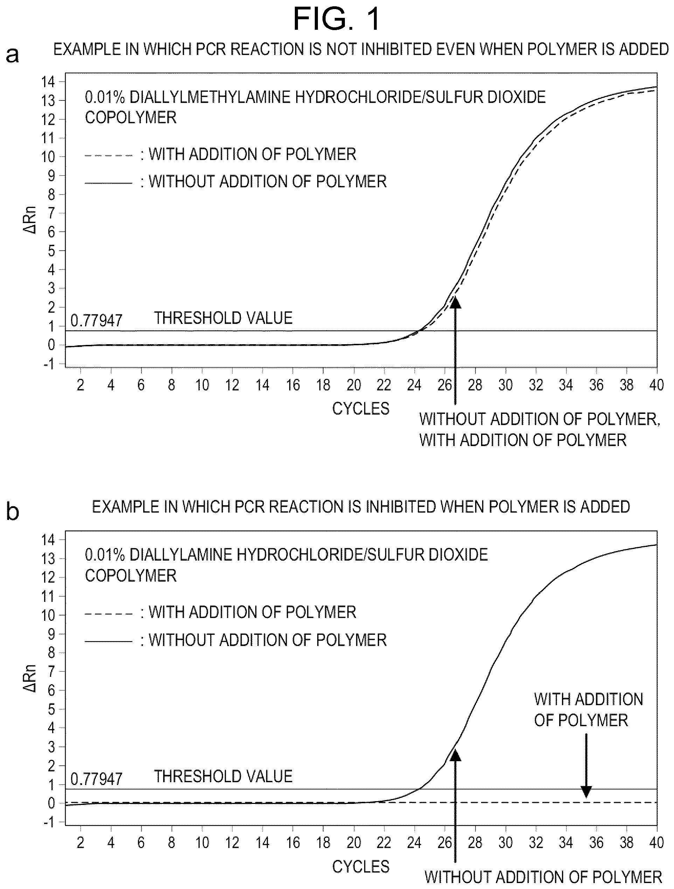

FIG. 1(a) shows a nucleic acid amplification curve in the case of using the polymer of Example 1 (diallylmethylamine hydrochloride/sulfur dioxide copolymer), as an example of a nucleic acid amplification curve in a case where a PCR reaction was not inhibited in Test 1. FIG. 1(b) shows a nucleic acid amplification curve in the case of using the polymer of Comparative Example 1 (diallylamine hydrochloride/sulfur dioxide copolymer), as an example of a nucleic acid amplification curve in a case where a PCR reaction was inhibited in Test 1.

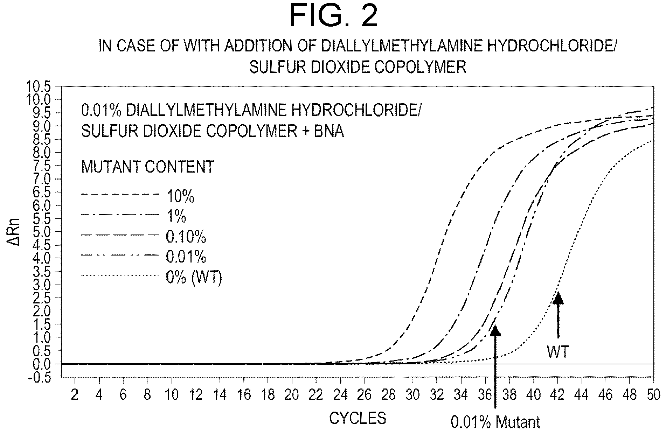

FIG. 2 shows a nucleic acid amplification curve in the case that KRAS genes containing 10%, 1%, 0.10%, 0.01%, or 0% (WT) of KRAS mutant genes were subjected to amplification by a real-time PCR reaction as a template DNA in the presence of a clamp nucleic acid containing BNA (BNA clamp) and the polymer of Example 1 (diallylmethylamine hehydrochloride/sulfur dioxide copolymer).

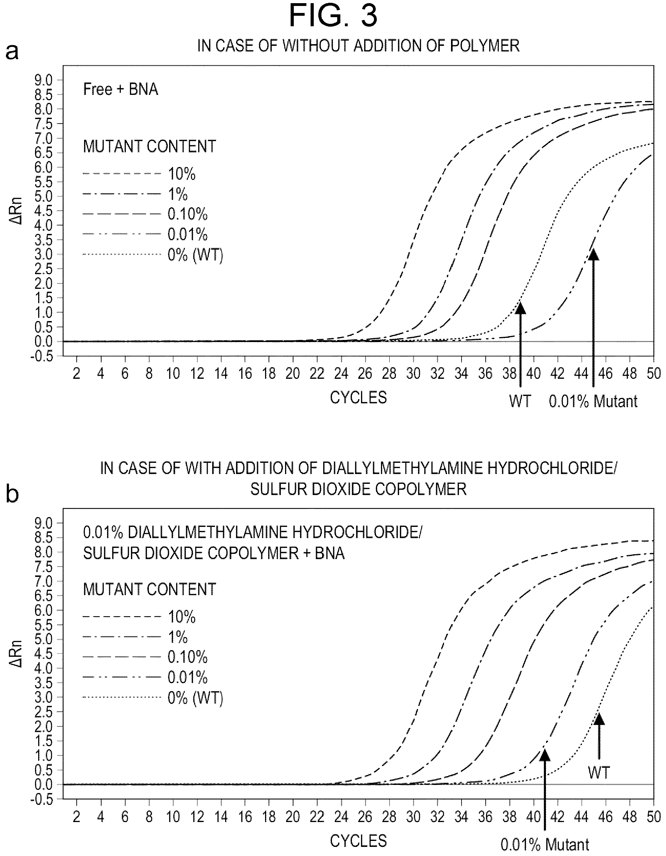

FIG. 3(a) shows a nucleic acid amplification curve in the case that BRAF gene-derived DNA containing 10%, 1%, 0.10%, 0.01%, or 0% (WT) of BRAF mutant gene-derived DNA was subjected amplification by a real-time PCR reaction as a template DNA in the presence of the BNA clamp but without adding the polymer. FIG. 3(b) shows a nucleic acid amplification curve in the case that BRAF gene-derived DNA containing 10%, 1%, 0.10%, 0.01%, or 0% (WT) of BRAF mutant gene-derived DNA was subjected to amplification by a real-time PCR reaction as a template DNA in the presence of the BNA clamp and the polymer of Example 1 (diallylmethylamine hydrochloride/sulfur dioxide copolymer).

### DESCRIPTION OF EMBODIMENTS

The invention is set out in the appended set of claims. Embodiments of the present invention will be described in detail below.

### 1. Method for Detecting Mutation in Target Base Sequence Relative to Standard Base Sequence

One embodiment of the present invention relates to a method for detecting mutations in a target base sequence of a nucleic acid in a sample relative to a standard base sequence, comprising the steps of:
subjecting the nucleic acid in the sample to a nucleic acid amplification reaction as a template with
a clamp nucleic acid having a base sequence complementary to the standard base sequence and containing at least one artificial nucleotide residue, and
a diallylamine/sulfur dioxide copolymer that comprises
a constituent unit (A) having a structure represented by general formula (I-a) or general formula (I-b), or a structure of an acid addition salt thereof: 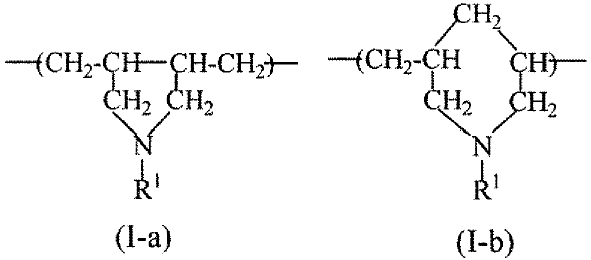
wherein R¹ represents an alkyl group having 1 to 10 carbon atoms, and
a constituent unit (B) having a structure represented by general formula (I-c): 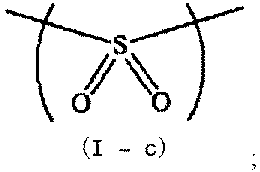 and
determining the presence of the mutation based on the total amount of nucleic acid amplification products obtained by the nucleic acid amplification reaction, the number of amplification cycles of the nucleic acid amplification reaction required for the total amount of the nucleic acid amplification products to reach a threshold value, the amount of nucleic acids with the mutation in the nucleic acid amplification products or the number of amplification cycles of the nucleic acid amplification reaction required for the amount of nucleic acids with the mutation in the nucleic acid amplification products to reach a threshold value.

### Sample

The samples provided for the methods are assumed to contain a nucleic acid for which the detection of mutation is desired or a nucleic acid that may be subject to amplification inhibition, depending on the embodiments. In the present embodiment, the sample is assumed to contain a nucleic acid for which the detection of mutation is desired. The sample is, for example, prepared from a specimen collected from a mammal, typically a human. Examples thereof include liquid samples such as blood, pleural effusion, bronchial lavage fluid, bone marrow fluid, and lymph fluid, and samples prepared from solid samples such as lymph nodes, blood vessels, bone marrow, brain, spleen, and skin. Since the method of the present embodiment can detect mutations in a nucleic acid with very high sensitivity, for example, not only a sample from a specimen collected from a lesion site such as cancer but also a sample from a specimen containing only a trace amount of mutant gene such as blood can be used.

### Target Base Sequence

As used herein, the "target base sequence" means a base sequence composed of one or more bases to be subjected to the detection of mutation or the inhibition of amplification, depending on the methods according to the embodiments. Further, the "nucleic acid in a sample" is a nucleic acid that may contain a base sequence to be subjected to the detection of mutation or the inhibition of amplification. However, the "target base sequence" is not understood to actually has mutations or contain a base sequence of which amplification is inhibited, and the "nucleic acid in a sample" is not understood to actually contain a base sequence that is a target for the detection of mutation or the inhibition of amplification.

In the method according to the present embodiment, the "target base sequence" is a base sequence composed of one or more bases which is a target for detecting the presence or absence of mutations, and the "nucleic acid in a sample" is a nucleic acid containing a base sequence that is a target for detecting presence or absence of mutations. The "region containing a target base sequence" is a region to be amplified by the methods of the present disclosure.

### Nucleic Acid in Sample

The nucleic acid may be DNA or RNA and may include any natural or artificial nucleic acids such as genomic DNA, cDNA, plasmid DNA, cell-free DNA, circulating DNA, RNA, miRNA, and mRNA. In the case of using a sample from a specimen collected from a mammal, typically a human, the nucleic acid to be subjected to detection of mutation is often DNA, typically genomic DNA having great clinical significance.

Examples of the genomic DNA include genomic DNA in which mutation (inherited or acquired mutation) is associated with development of a specific disease and/or therapeutic sensitivity. A large number of such diseases are known, and representative examples thereof include various cancers. Examples of the gene in which mutation is known to be associated with development of cancer and/or therapeutic sensitivity, include ABL/BCR fusion gene (chronic myelogenous leukemia), HER2 gene (breast cancer), EGFR gene (non-small cell lung cancer), c-KIT gene (gastrointestinal stromal tumor), KRAS gene (colon cancer, pancreatic cancer), BRAF gene (melanoma, colorectal cancer), PI3KCA gene (lung cancer, colorectal cancer), FLT3 gene (acute myelogenous leukemia), MYC gene (various cancers), MYCN gene (neuroblastoma), MET gene (Lung cancer, gastric cancer, melanoma), BCL2 gene (follicular B-lymphoma), and EML4/ALK fusion gene (lung cancer).

### Standard Base Sequence and Mutation

As used herein, a "standard base sequence" means a base sequence that serves as a standard when determining whether a "target base sequence" has mutations in a method for detecting mutations in the "target base sequence", and the terms "mutation", "a mutation" are "mutations" mean a state in which the "target base sequence" has some difference in base sequence due to substitution, insertion, deletion, inversion, duplication, translocation, or a combination of two or more thereof, relative to the "standard base sequence". In the specification, such "mutation", "a mutation" or "mutations" may be a difference in base sequence of 20% or less, a difference in base sequence of 10% or less, a difference in base sequence of 5% or less, or a difference in base sequence of 1% or less.

A "standard base sequence" can be selected from various base sequences according to the purpose of a test, but is typically a base sequence derived from a wild-type nucleic acid, and is preferably a base sequence derived from a wild-type gene of which mutation is known to be associated with development of a specific disease and/or therapeutic sensitivity. Therefore, a "mutant nucleic acid" is typically a nucleic acid having some difference in base sequence due to substitution, insertion, deletion, inversion, duplication, translocation, or a combination of two or more thereof relative to a base sequence derived from a wild-type nucleic acid, and is preferably a nucleic acid having such a difference in base sequence relative to a wild-type gene of which mutation is known to be associated with development of a specific disease and/or therapeutic sensitivity. For example, it is known that mutations at the 12th codon or the 13th codon in the second exon of the KRAS gene are associated with the response rate of cetuximab, a molecularly targeted drug. As to the BRAF gene, it is known that mutations at the 600th codon in exon 15 are associated with the response rate of molecularly targeted drugs, cetuximab and panitumumab.

Since a specific diallylamine/sulfur dioxide copolymer particularly enhances the effect of inhibiting nucleic acid amplification by a clamp nucleic acid, an expected ratio of a target base sequence containing mutations with respect to the total of a target sequence not containing the mutations (i.e., standard base sequence) and the target sequence containing the mutations, which are contained in a nucleic acid in the above-mentioned sample, may be less than 0.1%. The expected ratio may be 0.05% or less in a preferred embodiment, and may be 0.01% or less in a more preferred embodiment. Meanwhile, a proportion of a target base sequence with mutation could be clinically expected for each mutation.

### Clamp Nucleic Acid

As used herein, the "clamp nucleic acid" is an artificial nucleic acid composed of an oligonucleotide that has a base sequence complementary to a "base sequence" which is a target for inhibiting nucleic acid amplification and contains at least one artificial nucleotide. It is to be noted that the "base sequence" as a target may be a "standard base sequence" or may be a "target base sequence", depending on the embodiments. The "clamp nucleic acid" hybridizes with the "base sequence" as a target for inhibiting nucleic acid amplification to inhibit nucleic acid amplification, but does not strongly hybridize with a nucleic acid of a base sequence that is not complementary to the targeted base sequence and does not inhibit nucleic acid amplification.

In a method for detecting mutations in a "target base sequence", a "base sequence" of which nucleic acid amplification is inhibited by a "clamp nucleic acid" is a "standard base sequence", and the "clamp nucleic acid" hybridizes with the "standard base sequence " to inhibit its nucleic acid amplification.

Examples of the artificial nucleotide include a peptide nucleic acid (PNA), a bridged nucleic acid (BNA), a peptide nucleic acid having a phosphate group (PHONA), and a morpholino nucleic acid. The first generation BNA is also referred to as locked nucleic acid (LNA) (see, for example, Non-Patent Literatures 2 and 3 and Patent Literatures 1 and 2.).

A clamp nucleic acid containing PNA (PNA clamp) is a fully modified artificial nucleic acid composed of an oligonucleotide containing one or more nucleotides forming a backbone with a peptide bond instead of a phosphate bond of nucleic acid. A modified nucleotide typically has a structure in which a sugar-phosphate backbone of the nucleotide is replaced with a backbone having N-(2-aminoethyl)glycine as a unit, and bases are bonded through a methylenecarbonyl bond. The details of the PNA are as described in Non-Patent Literature 6. Since a PNA clamp has a stronger ability to hybridize with a DNA strand having a complementary structure than a natural DNA has and does not undergo degradation by a nucleolytic enzyme, the PNA clamp has desirable characteristics as a clamp nucleic acid.

A clamp nucleic acid containing BNA (BNA clamp) is an artificial nucleic acid composed of an oligonucleotide containing one or more artificial nucleotides having a structure in which an oxygen atom at the 2'-position and a carbon atom at the 4'-position of ribose are crosslinked, and the first generation BNA is also referred to as locked nucleic acid (LNA). The BNA clamp is such that a helical structure is fixed, and when hybridizing with a nucleic acid having a complementary strand, the BNA clamp can form a very stable double strand. The bonding to a nucleic acid having a complementary strand is strengthened in proportion to the number of modified nucleotides, and thus an appropriate hybridizing ability can be acquired by adjusting the length of the nucleic acid and the number of modified nucleotides, and it is possible to exhibit a clamping effect with a relatively short oligonucleotide chain.

Representative examples of the BNA clamp are oligonucleotides containing modified nucleotides having the following structures:

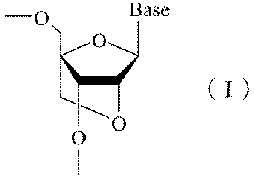

First generation BNA: 2',4'-BNA (LNA) (for example, Patent Literature 10) 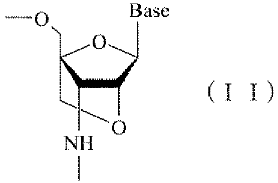 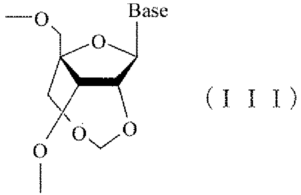 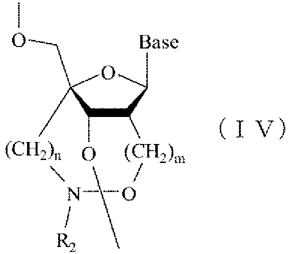 wherein
Base represents a pyrimidine or purine nucleic acid base, or a pyrimidine or purine nucleic acid base substituted with a substituent selected from the group consisting of a hydroxyl group, a hydroxyl group protected with a protective group for nucleic acid synthesis, an alkoxy group having 1 to 5 carbon atoms, a mercapto group, a mercapto group protected with a protective group for nucleic acid synthesis, an alkylthio group having 1 to 5 carbon atoms, an amino group, an amino group protected with a protective group for nucleic acid synthesis, an amino group substituted with an alkyl group having 1 to 5 carbon atoms, an alkyl group having 1 to 5 carbon atoms, and a halogen atom (for example, a fluorine atom, a chlorine atom, a bromine atom, or an iodine atom);
R₂ represents a hydrogen atom, a linear or branched alkyl group having 1 to 20 carbon atoms, a linear or branched alkenyl group having 2 to 20 carbon atoms, a cycloalkyl group having 3 to 10 carbon atoms, an aryl group having 6 to 14 carbon atoms, an aralkyl group in which an alkyl group having 1 to 6 carbon atoms is substituted with an aryl group having 6 to 14 carbon atoms, an acyl group, a sulfonyl group, a silyl group, or a labeling molecule;
m is an integer of 0 to 2; and
n is an integer of 1 to 3.

A modified nucleotide of formula (IV) is preferably such that the Base is selected from the group consisting of benzoylaminopurin-9-yl, adeninyl, 2-isobutyrylamino-6 hydroxypurin-9-yl, guaninyl, 2-oxo-4 benzoylamino-1,2-dihydropyrimidin-1-yl, cytosinyl, 2-oxo-5-methyl-4-benzoylamino-1,2-dihydropyrimidin-1-yl, 5-methylcytosinyl, uracinyl, and thyminyl groups, R₁ is a methyl group, m is 0, and n is 1.

However, the BNA used in the present disclosure is not limited to the above-described ones, and other BNAs (for example, 5'-amino-2',4'-BNA, 2',4'-BNACOC, and 2',4'-BNANC) can also be used. Further, in the BNA clamp, the bond between a modified nucleotide and a nucleotide or the bond between modified nucleotides is usually a phosphodiester bond, but may include a phosphorothioate bond. The details of the BNA and BNA clamp are described in Patent Literatures 1 to 10, and those skilled in the art to which the present disclosure is pertained can easily understand what artificial nucleic acid corresponds to the BNA clamp from known technical matters such as these documents.

As the clamp nucleic acid, a BNA clamp is preferable, and a BNA clamp containing a modified nucleotide having a structure of formula (IV) is particularly preferable, from the viewpoint that a relatively small number of nucleotides allows a stable double strand to be formed with a nucleic acid having a complementary sequence thereto to selectively inhibit nucleic acid amplification of the nucleic acid (ΔΔΔCt value is large with a method for detecting mutations based on the number of amplification cycles of a wild-type gene, such as an intercalator method that will be described later).

The length of a clamp nucleic acid and the number of the artificial nucleotides thereof are factors that determine the binding force to a nucleic acid that is subjected to inhibition of nucleic acid amplification. Therefore, it is preferable that these factors are appropriately set so that a clamp nucleic acid can strongly bind to a nucleic acid that is subjected to inhibition of nucleic acid amplification (in the method for detecting mutations, a nucleic acid having the standard base sequence) and selectively inhibit amplification of the nucleic acid. From this point of view, the length of a clamp nucleic acid is usually 5- to 50-mer, preferably 6- to 30-mer, more preferably 7- to 25-mer, even more preferably 8- to 20-mer, and particularly preferably 9-to 15-mer.

From the same point of view, the proportion of the artificial nucleic acids in a clamp nucleic acid is usually 10 to 100%, preferably 30 to 95%, more preferably 40 to 90%, even more preferably 50 to 85%, and particularly preferably 60 to 80%.

The synthesis of a clamp nucleic acid can be performed according to the disclosure in literature such as Patent Literatures 7 to 10. Further, the synthesis of a clamp nucleic acid may be outsourced to a contractor who synthesizes the clamp nucleic acids.

### Diallylamine/Sulfur Dioxide Copolymer

In the method of the present disclosure, when subjecting a nucleic acid in a sample to a nucleic acid amplification reaction as a template, the nucleic acid amplification reaction is performed in a state where a diallylamine/sulfur dioxide copolymer having a specific structure is present together with the above-described clamp nucleic acid. The diallylamine/sulfur dioxide copolymer used in the present disclosure per se does not inhibit a nucleic acid amplification reaction but enhances a selective nucleic acid amplification inhibitory effect by a clamp nucleic acid, and comprises a constituent unit (A) having a structure represented by general formula (I-a) or general formula (I-b), or a structure of an acid addition salt thereof: 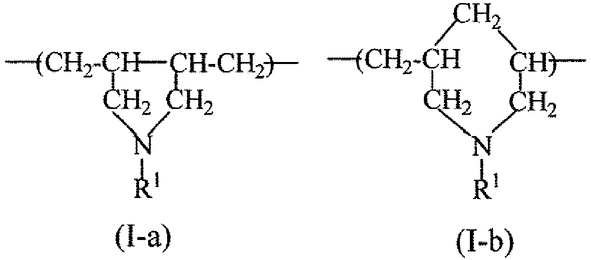
wherein R¹ represents an alkyl group having 1 to 10 carbon atoms, and
a constituent unit (B) having a structure represented by general formula (I-c):

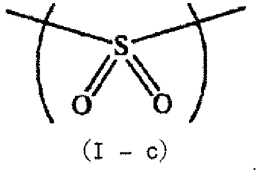

The diallylamine/sulfur dioxide copolymer is a copolymer comprising the above-described constituent unit (A) and the above-described constituent unit (B), and the copolymer may consist of a constituent unit (A) and a constituent unit (B), or may comprise another constituent unit in addition to a constituent unit (A) and a constituent unit (B). Examples of the other constituent unit include various cationic constituent units other than a constituent unit (A), various nonionic constituent units other than a constituent unit (B), and various anionic constituent units.

The proportion of the total of a constituent unit (A) and a constituent unit (B) in the whole constituent units of the diallylamine/sulfur dioxide copolymer is not particularly limited, but is usually 80 mol% or more, preferably 80 to 100 mol%, more preferably 90 to 100 mol%, and particularly preferably 95 to 100 mol%.

### Constituent Unit (A)

A constituent unit (A) constituting a diallylamine/sulfur dioxide copolymer used in the present disclosure is a diallylamine-based constituent unit having the above-described specific structure.

The diallylamine/sulfur dioxide copolymer may comprise only one type of constituent unit (A) or may contain two or more types of constituent units (A). When two or more types of constituent units (A) are comprised, the proportion of the total of a constituent unit (A) and a constituent unit (B) in the whole constituent units of a diallylamine/sulfur dioxide copolymer, and the proportion of a constituent unit (A) and a constituent unit (B) are calculated based on the total of the two or more types of constituent units (A).

A constituent unit (A) has a structure represented by general formula (I-a) or general formula (I-b), or a structure of an acid addition salt thereof:

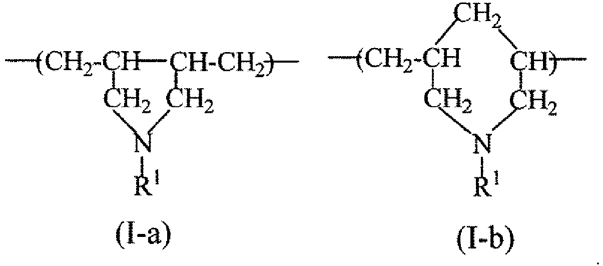

In general formula (I-a) and general formula (I-b), R¹ represents an alkyl group having 1 to 10 carbon atoms.

In all of the options, since R¹ is a group having at least one carbon atom, a constituent unit (A) is a constituent unit having a structure containing a tertiary amino group.

R¹ is preferably an alkyl group having 5 or fewer carbon atoms, more preferably a methyl group or an ethyl group, and particularly preferably a methyl group.

A constituent unit (A) may have a structure that is an inorganic acid salt, an organic acid salt, or the like of the structure represented by the above-described structural formula (1-a) or (1-b), that is, a structure of an acid addition salt.

In the production of a diallylamine/sulfur dioxide copolymer, it is preferable to introduce a constituent unit (A) using an addition salt-type diallylamine monomer, from the viewpoint of production cost and the like. A process of removing an addition salt such as HCl from a polymer is complicated and causes an increase in cost, and it is thus preferable from the viewpoint of cost and the like to use an addition salt-type constituent unit (A) which can be produced without requiring such a process.

From the viewpoint of the ease of availability, controllability of reaction, and the like, the inorganic acid salt or organic acid salt in a constituent unit (A) of this embodiment is preferably a hydrochloride, a carboxylate, a sulfonate, or an alkyl sulfate, and particularly preferably a hydrochloride.

As shown in the name of the diallylamine/sulfur dioxide copolymer, the constituent unit (A) is usually derived from a diallylamine-based monomer.

More specific examples of a suitable constituent unit (A) include a constituent unit derived from diallylmethylamine and a hydrochloride thereof, and a constituent unit derived from diallylethylamine and a hydrochloride thereof.

The proportion of a constituent unit (A) in the whole constituent units of a diallylamine/sulfur dioxide copolymer is usually 40 mol% or more, preferably 50 to 80 mol%, and particularly preferably 50 to 70 mol%. The above-described proportion in a case of comprising two or more types of constituent units (A) is defined based on the total of the two or more types of constituent units (A).

### Constituent Unit (B)

A constituent unit (B) constituting a diallylamine/sulfur dioxide copolymer used in the present disclosure is a constituent unit having a structure represented by general formula (I-c):

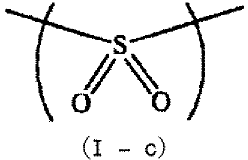

With regard to methods and kits and inhibition of nucleic acid amplification by a clamp nucleic acid according to the present disclosure, a polymer consisting of a constituent unit (A) inhibits a PCR reaction by its strong cationic charge, and the bond between the nucleic acids constituting a double strand is strengthened, whereas in a diallylamine/sulfur dioxide copolymer having a constituent unit (B) in addition to a constituent unit (A), the cationic charge of the constituent unit (A) is moderately interfered by the constituent unit (B). As a result, mutant genes can be detected at lower mutant gene content compared to the detection limit of mutant genes by the ordinary clamping methods, and the amplification of a base sequence that is subjected to a nucleic acid amplification reaction can be selectively inhibited.

As shown in the name of a diallylamine/sulfur dioxide copolymer, a constituent unit (B) is usually derived from sulfur dioxide.

The proportion of a constituent unit (B) in the whole constituent units of the diallylamine/sulfur dioxide copolymer is preferably 10 mol% or more.

The proportion of a constituent unit (B) is more preferably 20 to 50 mol%, and particularly preferably 30 to 50 mol%.

The ratio between a constituent unit (A) and a constituent unit (B) in a diallylamine/sulfur dioxide copolymer is also not particularly limited, and can be appropriately set in view of the purpose of the present disclosure, and the ratio can be set to any proportion as long as copolymerization is made possible. From the viewpoint of increasing the molecular weight of a diallylamine/sulfur dioxide copolymer, the proportion of both the constituent units is preferably not significantly different, and for example, the molar ratio of a constituent unit (A), as described above and a constituent unit (B), as described above is preferably 90 : 10 to 40 : 60, more preferably 80 : 20 to 50 : 50, and particularly preferably 70 : 30 to 50 : 50.

In a case where the structures of a constituent unit (A), as described above, are known, the molar ratio of a constituent unit (A), as described above, and a constituent unit (B), as described above, in a diallylamine/sulfur dioxide copolymer can be specified by reprecipitating the diallylamine/sulfur dioxide copolymer with an organic solvent such as isopropyl alcohol, or acetone, and by analyzing the reprecipitation product in appropriate modes according to the structures of the constituent units, using Perkin Elmer 2400II CHNS/O fully automatic elemental analyzer or an apparatus with a performance equivalent thereto. In addition, the measurement can be performed by using helium gas as a carrier gas, weighing a solid sample into a tin capsule, dropping the sample into a combustion tube to burn the sample at a combustion temperature of 1800°C or higher in pure oxygen gas, detecting each component of interest by a frontal chromatography system with a separation column and a thermal conductivity detector, and quantifying the content of each element using a calibration factor. Further, when the structure of a constituent unit (A), as described above, in a diallylamine/sulfur dioxide copolymer is unknown, the structure of a constituent unit (A), as described above, is specified by a known method with 1H-NMR or 13C-NMR prior to the above-mentioned measurement by the elemental analyzer. The molar ratio can also be calculated from the amount of each monomer supplied in the production (copolymerization) of a diallylamine/sulfur dioxide copolymer, and the amount of each monomer remaining without being incorporated into the diallylamine/sulfur dioxide copolymer. In this regard, the proportion (molar ratio) of the constituent unit derived from each monomer in the diallylamine/sulfur dioxide copolymer is considered almost corresponding to the monomer composition (molar ratio) for preparation of the constituent units, the mixing ratio of a monomer may be treated as the proportion (molar ratio) of a corresponding constituent unit for convenience herein.

When the above-described matters are put together, as the diallylamine/sulfur dioxide copolymer, a diallylmethylamine/sulfur dioxide copolymer, a diallylmethylamine acid addition salt/sulfur dioxide copolymer, a diallylethylamine/sulfur dioxide copolymer, and a diallylethylamine acid addition salt/sulfur dioxide copolymer are preferable, and a diallylmethylamine/sulfur dioxide copolymer and a diallylmethylamine acid addition salt/sulfur dioxide copolymer are particularly preferable.

A diallylamine/sulfur dioxide copolymer may have another constituent unit in addition to a constituent unit (A), as described above, and a constituent unit (B), as described above.

Examples of the other constituent unit include various cationic constituent units other than a constituent unit (A), as described above, various nonionic constituent units other than a constituent unit (B), as described above, and various anionic constituent units.

### (C) Cationic Constituent Units Other Than Constituent Unit (A)

Examples of various cationic constituent units (C) other than a constituent unit (A), as described above, include a diallylamine constituent unit other than a constituent unit (A), as described above, for example, a diallylamine constituent unit having a secondary or quaternary amino group or an addition salt thereof, various monoallylamine-based constituent units or addition salts thereof, and a cationic (meth)acrylamide-based constituent unit.

Among them, a diallylamine constituent unit having a secondary amino group can be preferably used.

When a diallylamine/sulfur dioxide copolymer has a cationic constituent unit other than the constituent unit (A), as described above, the content of the cationic constituent unit is not particularly limited, and a suitable amount thereof varies depending on the type of the cationic constituent unit, but the content is preferably 20 mol% or less, and particularly preferably 0 to 10 mol%.

### (D) Anionic Constituent Unit

An anionic constituent unit (D) that can be introduced as a constituent unit other than the constituent units (A) and (B), as described above, may be a constituent unit having an anionic group such as a carboxyl group and is not otherwise particularly limited, but preferred examples thereof include a constituent unit derived from an unsaturated dicarboxylic acid-based monomer such as maleic acid or fumaric acid, and a constituent unit derived from an unsaturated monocarboxylic monomer such as acrylic acid or methacrylic acid. Among them, maleic acid and acrylic acid can be preferably used.

When a diallylamine/sulfur dioxide copolymer has an anionic constituent unit, the content of an anionic constituent unit other than a constituent unit (B), as described above, is not particularly limited, and a suitable amount thereof varies depending on the type of the anionic constituent unit, but the content is preferably 20 mol% or less, and particularly preferably 0 to 10 mol%.

From the viewpoint of adjusting the cationic charge of a constituent unit (A), as described above, an anionic constituent unit (D) is preferably contained within the above-described range when the cationic charge of a constituent unit (A), as described above, is excessive, but an anionic constituent unit (D) is preferably not contained when the cationic charge of a constituent unit (A), as described above, is moderate.

### (E) Nonionic Constituent Unit Other Than Constituent Unit (B)

A nonionic constituent unit (E) other than a constituent unit (B) according to the present embodiment may be a constituent unit derived from a nonionic monomer other than sulfur dioxide, which can be copolymerized with a constituent unit (A), as described above, and an anionic constituent unit (B), as described above, and is not otherwise particularly limited. In this regard, constituent units derived from a methacrylic acid ester-based monomer, an acrylic acid ester-based monomer, a methacrylamide-based monomer, an acrylamide-based monomer, and the like can be preferably used. More specific examples include constituent units derived from methyl methacrylate, ethyl methacrylate, butyl methacrylate, methyl acrylate, ethyl acrylate, butyl acrylate, methacrylamide, N-methyl methacrylamide, dimethylmethacrylamide, N-(3-dimethylaminopropyl)methacrylamide, acrylamide, dimethylacrylamide, hydroxyethyl acrylamide, dimethylaminopropyl acrylamide, a dimethylaminopropyl acrylamide methyl chloride quaternary salt, acryloylmorpholine, isopropyl acrylamide, 4-t-butylcyclohexyl acrylate, and the like. Among them, it is particularly preferable to use a constituent unit derived from acrylamide, hydroxyethylacrylamide, or dimethylacrylamide.

A nonionic constituent unit other than a constituent unit (B), as described above, can usually be introduced into a polymer by using a nonionic monomer as a monomer.

When an diallylamine/sulfur dioxide copolymer has a nonionic constituent unit other than a constituent unit (B), as described above, the content of the nonionic constituent unit other than the constituent unit (B), as described above, is not particularly limited, and a suitable amount thereof varies depending on the type of the nonionic constituent unit, but the content is preferably 20 mol% or less, and particularly preferably 0 to 10 mol%.

The molecular weight of a diallylamine/sulfur dioxide copolymer is not particularly limited, and a diallylamine/sulfur dioxide copolymer having an appropriately suitable molecular weight may be obtained or polymerized in relation to the use form in a method, kit, and the like of the present disclosure, other components, and the like.

From the viewpoint of the ease of forming a reaction mixture with a nucleic acid or the like and performing polymerization in a practically acceptable time and cost, the weight average molecular weight (Mw) of a diallylamine/sulfur dioxide copolymer is preferably 300 to 30000. The weight average molecular weight (Mw) is more preferably 1000 to 9000. The weight average molecular weight (Mw) is even more preferably 1500 to 6000, and particularly preferably 2000 to 4000.

The weight average molecular weight (Mw) of a diallylamine/sulfur dioxide copolymer can be measured by, for example, gel permeation chromatography (GPC method) using a liquid chromatograph.

Specifically, for example, the weight average molecular weight can be measured by the following procedure with a Hitachi L-6000 high performance liquid chromatograph. Hitachi L-6000 is used as an eluent channel pump, a Shodex RI-101 differential refractive index detector is used as a detector, and Shodex Asahipak water-based gel filtration type GS-220HQ (exclusion limit molecular weight 3000) and GS-620HQ (exclusion limit molecular weight 2 million) connected in series are used as columns. A sample is prepared at a concentration of 0.5 g/100 ml with an eluent, and 20 µL thereof is used. As an eluent, a 0.4 mol/liter sodium chloride aqueous solution is used. The column temperature is 30°C and the flow rate is 1.0 ml/min. A calibration curve is obtained using polyethylene glycols having molecular weights of 106, 194, 440, 600, 1470, 4100, 7100, 10300, 12600, 23000, and the like as standard materials, and the weight average molecular weight (Mw) of a polymer is obtained on the basis of the calibration curve.

The molecular weight of a diallylamine/sulfur dioxide copolymer can be appropriately adjusted by adjusting the type and composition of monomers subjected to polymerization, the temperature, time, and pressure in the polymerization step, the type and amount of a radical initiator and the like used in the polymerization step, and the like.

The rotational viscosity [η] of a diallylamine/sulfur dioxide copolymer is also not particularly limited, and can be appropriately set in consideration of the use form, production cost, and the like in a method, kit, and the like of the present disclosure, but the rotational viscosity [η] is preferably 1.0 to 150.0 mPa·s (25°C), more preferably 1.5 to 30.0 mPa·s (25°C), and particularly preferably 2.0 to 20.0 mPa·s (25°C), in 25.0% by mass of aqueous solution.

The rotational viscosity [η] can be measured by any method commonly used in the art, and can be measured by, for example, a digital B-type viscometer DV-3T manufactured by AMETEK Brookfield, Inc. The measurement can be performed using a ULA adaptor, typically at a liquid volume of 16 mL and a liquid temperature of 25°C.

The rotational viscosity [η] can also be appropriately adjusted by adjusting the dilution concentration during polymerization, the type and composition of the monomers contributing to polymerization, the temperature, time, and pressure in the polymerization step, the type and amount of a radical initiator and the like used in the polymerization step, and the like.

### Method for Producing Diallylamine/Sulfur Dioxide Copolymer

The method for producing a diallylamine/sulfur dioxide copolymer is not particularly limited, and the diallylamine/sulfur dioxide copolymer can be produced by a method conventionally known in the art, but for example, the copolymer can be produced by copolymerizing a diallylamine-based monomer having a structure corresponding to a constituent unit (A), a monomer that corresponds to the constituent unit (B), which is usually sulfur dioxide, and a monomer having a structure corresponding to another constituent unit as desired.

The solvent used in copolymerizing a diallylamine-based monomer, sulfur dioxide, and others is not particularly limited, and may be a water-based solvent or an organic solvent such as alcohol, ether, sulfoxide, or amide, but the solvent is preferably a water-based solvent.

The monomer concentration in the case of copolymerizing a diallylamine-based monomer, sulfur dioxide, and others varies depending on the types of monomers and depending on the type of a solvent in which copolymerization is performed, but the monomer concentration is usually 10 to 75% by mass in a water-based solvent. This copolymerization reaction is usually a radical polymerization reaction, and is performed in the presence of a radical polymerization catalyst. The type of the radical polymerization catalyst is not particularly limited, and preferred examples of the radical polymerization catalyst include a peroxide such as t-butyl hydroperoxide, a persulfate such as ammonium persulfate, sodium persulfate, or potassium persulfate, and a water-soluble azo compound such as an azobis-based compound, or a diazo-based compound.

The amount of a radical polymerization catalyst to be added is commonly 0.1 to 20 mol%, and preferably 1.0 to 10 mol% in relation to the whole monomers. The polymerization temperature is commonly 0 to 100°C and preferably 5 to 80°C, and the polymerization time is commonly 1 to 150 hours and preferably 5 to 100 hours. As to the polymerization atmosphere, even in the air, there is no major problem in polymerization, and the polymerization can be performed in an atmosphere of an inert gas such as nitrogen.

### Nucleic Acid Amplification Reaction

In a method according to the present embodiment, a nucleic acid amplification reaction with a nucleic acid in a sample as a template is performed with a clamp nucleic acid and a specific diallylamine/sulfur dioxide copolymer.

The method for nucleic acid amplification is not particularly limited, and examples thereof include PCR methods, such as a real-time PCR method, a digital PCR method, a traditional PCR method, and an RT-PCR method, a LAMP method, an ICAN method, a NASBA method, an LCR method, an SDA method, a TRC method, and a TMA method. The method for nucleic acid amplification is preferably a PCR method from the viewpoint of versatility and convenience of the technique, and is more preferably a real-time PCR method from the viewpoint of easy quantification of nucleic acid amplification products.

The composition of a reaction mixture for nucleic acid amplification varies somewhat depending on the method for nucleic acid amplification used, but usually, the mixture may comprise a primer set, deoxynucleoside triphosphate (for example, dATP, dTTP, dCTP, and dGTP; hereinafter collectively referred to as dNTPs.) as substrates, and a nucleic acid synthase (for example, DNA polymerase) in a buffer in addition to the clamp nucleic acid and the diallylamine/sulfur dioxide copolymer described above, and may also comprise, in some cases, magnesium ions (for example, contained at a concentration of 1 to 6mM in the reaction mixture) as a cofactor of the nucleic acid synthase.

Further, in the case of a method for nucleic acid amplification that simultaneously performs nucleic acid amplification and detection of amplified nucleic acid, such as a real-time PCR method, an appropriate detection reagent such as an intercalator, a fluorescent-labeled probe, or a cycling probe is also contained in a reaction mixture for nucleic acid amplification.

The primer set is only required to be able to amplify a region containing a target base sequence by a method for nucleic acid amplification, and is usually designed to hybridize to a predetermined region adjacent to a region to be amplified, but the primer may contain a sequence that hybridizes to a part of the target base sequence.

The primer can be composed of a nucleic acid such as DNA or RNA. Regarding the concentration of the primer, an appropriate concentration may be usually selected in the range of 20nM to 2µM in a reaction mixture. The length of the primer is usually 5- to 40-mer, preferably 12- to 35-mer, more preferably 14- to 30-mer, and even more preferably 15- to 25-mer. The distance between primers, that is, the region to be amplified, is usually 50 to 5000 bases, and preferably 100 to 2000 bases. The primers may be designed manually, or an appropriate primer design software may be used. Examples of such software include Primer3 software (http://frodo.wi.mit.edu).

The concentration of dNTPs may be selected from concentrations at which the concentration of each of dATP, dTTP, dCTP, and dGTP in a reaction mixture is usually in the range of 100 to 400 µM. Examples of the nucleic acid synthase include DNA polymerase, RNA polymerase, and reverse transcriptase, and an enzyme having properties suitable for the method for nucleic acid amplification may be used. For example, in the case of a PCR method, TaqDNA polymerase, PfuDNA polymerase, and other various heat-resistant DNA polymerases developed by bioscience-related companies are preferably used.

It is preferable to select a buffer having an optimum pH and an optimum salt concentration for a DNA polymerase to be used. Depending on the method for nucleic acid amplification, ribonuclease H (RNaseH), reverse transcriptase (RT), or the like may be further contained. Since various kits are commercially available for each type of methods for nucleic acid amplification, a reaction mixture for nucleic acid amplification may be prepared with such a commercially available kit.

The concentration of a nucleic acid that serves as a template for a nucleic acid amplification reaction, i.e. a "nucleic acid in sample", in a reaction mixture may be usually 0.3 ng to 3 µg per 100 µl of the reaction mixture, but when the amount of a nucleic acid as a template is too large, the frequency of non-specific amplification increases, and therefore, it is preferable to suppress the concentration to 0.5 µg or less per 100 µl of the reaction mixture.

Regarding the amount of a clamp nucleic acid in a reaction mixture, it is preferable to select such an amount that, in a case where a target base sequence has mutations, amplification of a nucleic acid having a standard base sequence is sufficiently inhibited and a nucleic acid containing the target base sequence with the mutations is preferentially amplified, according to the amount of the nucleic acid having the target base sequence assumed to have mutations with respect to the amount of the nucleic acid having the standard base sequence (for example, a wild-type nucleic acid) in a sample. Since the majority of a wild-type nucleic acid is usually found in a sample with respect to a mutant nucleic acids, the amount is preferably set to 10nM to 1µM, more preferably 20nM to 500nM, and particularly preferably 50nM to 200nM.

Regarding the concentration of a diallylamine/sulfur dioxide copolymer in a reaction mixture, it is preferable to select an appropriate concentration so that the effect of selectively inhibiting nucleic acid amplification reaction by a clamp nucleic acid can be enhanced without adversely affecting the nucleic acid amplification reaction. The concentration of a diallylamine/sulfur dioxide copolymer in a reaction mixture varies depending on the type of the polymer, but from the above viewpoint, the concentration may be usually 0.001 to 0.500% by mass, and is preferably 0.005 to 0.100% by mass, more preferably 0.010 to 0.050% by mass, and particularly preferably 0.020 to 0.030% by mass.

When a nucleic acid amplification reaction with a nucleic acid in a sample as a template is performed with a clamp nucleic acid and a diallylamine/sulfur dioxide copolymer described above, the clamp nucleic acid strongly binds to a nucleic acid having a standard base sequence and inhibits annealing of a primer and/or extension of the primer during the amplification reaction, and the coexisting diallylamine/sulfur dioxide copolymer enhances this inhibitory effect. On the other hand, the binding force of the clamp nucleic acid is greatly reduced for a base sequence having mutations relative to the standard base sequence, annealing and primer extension are hardly inhibited, and a normal nucleic acid amplification reaction occurs. Further, a diallylamine/sulfur dioxide copolymer itself does not inhibit a nucleic acid amplification reaction. As a result, according to a method of the present embodiment, even in a case where nucleic acids having mutations among the nucleic acids in a sample are in a trace amount (for example, in the case of 0.1% or less or 0.05% or less, particularly 0.02% or less), and a nucleic acid having a standard base sequence is dominant, amplification of the nucleic acid having the standard base sequence is selectively inhibited, and the nucleic acids having mutations are preferentially amplified, whereby nucleic acids having mutations, which are included in a trace amount, can be detected.

According to the method of the present embodiment, the presence of mutation is detected employing the above-mentioned characteristics of the nucleic acid amplification reaction. That is, amplification of a nucleic acid having a standard base sequence is selectively inhibited. whereas in case that a target base sequence has mutations relative to the standard base sequence, a region containing the target base sequence is preferentially amplified, and as a result, differences occur in the progress rate of the nucleic acid amplification reaction, the amount of the reaction product, and the like depending on the presence or absence of mutations. Therefore, these differences are used for the detection of mutations.

In the present embodiment, the detection of mutation may be done by a method of detecting the final product of an amplification reaction and a method of monitoring amplification over time during a amplification reaction. For example, mutation can be detected based on the total amount of the obtained nucleic acid amplification products, the number of amplification cycles of a nucleic acid amplification reaction required for the total amount of the nucleic acid amplification products to reach a threshold value, the amount of nucleic acids having mutation in nucleic acid amplification products, or the number of amplification cycles of a nucleic acid amplification reaction required for the amount of nucleic acids having mutation in nucleic acid amplification products to reach a threshold value. Particularly, from the viewpoint of simplicity of implementation and a wide adaptation range, preferable is a method of detecting mutation based on the number of amplification cycles of a nucleic acid amplification reaction required for the total amount of nucleic acid amplification products to reach a threshold value.

In the meantime, accurate and highly sensitive detection can be achieved by examining the base sequences of amplification products by a direct sequencing method or the like. However, as demonstrated in Examples described below, according to the method of the present embodiment, it is possible to accurately detect mutation with high sensitivity without depending on the sequence determination.

Examples of a method for detecting mutation based on the total amount of nucleic acid amplification products include: a method which comprises subjecting a solution after an amplification reaction to deproteinization treatment with a phenol/chloroform (1 : 1) solution, subsequently purifying the aqueous layer directly or after ethanol precipitation using an appropriate purification kit, and measuring the absorbance of the purified solution at a wavelength of 260 nm using a spectrophotometer; a method which comprises subjecting the amplification products to electrophoresis with an agarose gel or a polyacrylamide gel and then detecting mutation with an appropriate probe by a Southern hybridization method; a method of detecting mutation by a chromatography hybridization method using gold nanoparticles; a method of measuring turbidity of a solution containing amplified nucleic acids; and a method which comprises fluorescently labeling an amplification primer at the 5'-end in advance, performing an amplification reaction followed by electrophoresis with an agarose gel or a polyacrylamide gel, subsequently introducing the fluorescence in an imaging plate, and detecting it using an appropriate detection apparatus.

Examples of a method for detecting mutation based on the number of amplification cycles of a nucleic acid amplification reaction required for the total amount of nucleic acid amplification products to reach a threshold value, include real-time PCR methods such as an intercalator method, a TaqManTM probe method, and a cycling probe method. The threshold value can be automatically detected by a real-time PCR apparatus (for example, Step One Plus real-time PCR system, manufactured by Thermo Fisher Scientific, Inc.).

The intercalator method is a method with an intercalating fluorescent dye (also referred to as an intercalator). An intercalator is a reagent that is specifically introduced between a pair of bases of double-stranded nucleic acids to emit fluorescence, and it emits fluorescence when irradiated with excitation light. Based on detection of the fluorescence intensity originating from an intercalator, the amount of primer extension products can be found. According to the present embodiment, monitoring this fluorescence intensity in real time makes it possible to know the number of amplification cycles of a nucleic acid amplification reaction required for the total amount of nucleic acid amplification products to reach a threshold value. According to the present embodiment, any intercalator usually used in this field can be used, and examples thereof include SYBRTM Green I (Molecular Probes, Inc.), ethidium bromide, and fluorene.

The TaqManTM probe method is a method in which a trace amount of nucleic acid of interest can be quantitatively detected with high sensitivity by a real-time PCR method using an oligonucleotide probe, wherein the probe is labeled with a fluorescent group (reporter) at one end (usually at the 5'-end) and a quenching group at the other end (usually at the 3'-end) and hybridizes to a specific region of a target nucleic acid. With the probe, fluorescence of the reporter is inhibited by the quenching group in a normal state. In a condition in which this fluorescent probe is completely hybridized with a detection region, PCR starts from the outside of the region using DNA polymerase. As an extension reaction by the DNA polymerase proceeds, the fluorescent probe is hydrolyzed by the exonuclease activity of the DNA polymerase, the reporter dye is released, and fluorescence is emitted. According to the present embodiment, monitoring this fluorescence intensity in real time make it possible to know the number of amplification cycles of a nucleic acid amplification reaction required for the total amount of nucleic acid amplification products to reach a threshold value.

The cycling probe method is a highly sensitive detection method with a combination of RNase H and a chimeric probe composed of RNA and DNA. The probe is labeled with a fluorescent substance (reporter) on one side and a substance that quenches fluorescence (quencher) on the other side across the RNA portion. This probe does not emit fluorescence due to quenching in an intact state, but once the sequence forms a hybrid with a complementary amplification product, the RNA portion is cleaved by RNase H and the probe emits strong fluorescence. According to the present embodiment, the total amount of nucleic acid amplification products can be known by measuring this fluorescence intensity, and monitoring the fluorescence intensity in real time makes it possible to know the number of amplification cycles of a nucleic acid amplification reaction required for the total amount of nucleic acid amplification products to reach a threshold value.

In this method, when there is a mismatch in the vicinity of the RNA of a cycling probe, cleavage by RNase H does not occur, and this method therefore makes possible highly specific detection which is capable of recognizing even a difference in a single base. The cycling probe can also be acquired by outsourcing its design and synthesis to an appropriate company, or the like.

Examples of a method for detecting mutation based on the amount of nucleic acid with base mutation in nucleic acid amplification products include a Sanger sequencing method and a melting curve analysis method.

Examples of a method for detecting mutation based on the number of amplification cycles of a nucleic acid amplification reaction required for the amount of nucleic acid with base mutation in nucleic acid amplification products to reach a threshold value, include a real-time PCR method with a probe that specifically binds to a nucleic acid with base mutation.

### 2. Kit for Detecting Mutation in Target Base Sequence

In another embodiment, the present invention provides a kit for detecting mutation in a target base sequence of a nucleic acid in a sample relative to a standard base sequence comprising:
a clamp nucleic acid having a base sequence complementary to the standard base sequence and containing at least one artificial nucleotide residue;
a diallylamine/sulfur dioxide copolymer that comprises
a constituent unit (A) having a structure represented by general formula (I-a) or general formula (I-b), or a structure of an acid addition salt thereof: 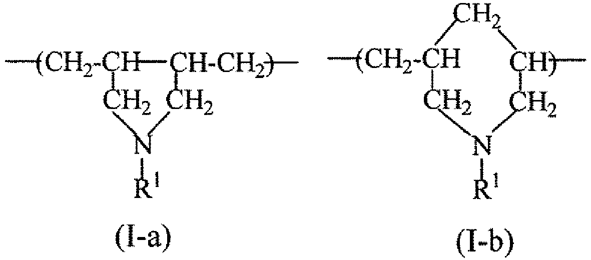
wherein R¹ represents an alkyl group having 1 to 10 carbon atoms, and
a constituent unit (B) having a structure represented by general formula (I-c): 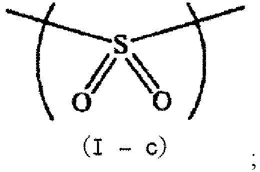
optionally a primer set capable of amplifying a region containing a target base sequence by a nucleic acid amplification method; and
optionally other reagents for implementing a nucleic acid amplification reaction.

The details of the clamp nucleic acid, the diallylamine/sulfur dioxide copolymer, and the primer set are as described in the method for detecting mutation in a target base sequence, and preferred embodiments and specific examples thereof are the same as those of the method.

Other reagents for implementing a nucleic acid amplification reaction include nucleoside triphosphates, nucleic acid synthases, and buffer solutions for amplification reaction. The nucleoside triphosphates may include substrates according to nucleic acid synthases (dNTP, rNTP, and the like). The nucleic acid synthases are enzymes according to the nucleic acid amplification method conducted with the kit, and examples thereof include DNA polymerase, RNA polymerase, and reverse transcriptase. Examples of the buffer solutions for amplification reaction include buffer solutions used in implementing a conventional nucleic acid amplification reaction or hybridization reaction, such as a Tris buffer solution, a phosphate buffer solution, a veronal buffer solution, a borate buffer solution, and a Good's buffer solution. The pH thereof is not particularly limited, but a pH in the range of 5 to 9 is usually preferable.

A kit according to the present embodiment may also comprise a reagent for detecting the above-described nucleic acid amplification reaction products. Examples thereof include an intercalator, a fluorescent-labeled probe, and a cycling probe, which are used for real-time PCR.

Further, other components generally used as nucleic acid amplification reaction reagents, such as a stabilizer and a preservative, may also be comprised.

A primer set and other reagents for implementing a nucleic acid amplification reaction are necessary for implementing a nucleic acid amplification reaction, but may not be comprised in the kit. Other components such as a reagent for detecting a nucleic acid amplification reaction product, a stabilizer, and a preservative are optional components for nucleic acid amplification reactions, and may be not comprised in the kit.

### 3. Method for Inhibiting Amplification of Nucleic Acid Having Predetermined Target Base Sequence in Nucleic Acid Amplification Reaction

In still another embodiment, the present invention provides a method for inhibiting amplification of a nucleic acid in a sample with a predetermined target base sequence in a nucleic acid amplification reaction the method comprising
subjecting the nucleic acid to a nucleic acid amplification reaction as a template with:
a clamp nucleic acid having a base sequence complementary to the target base sequence and containing at least one artificial nucleotide residue, and
a diallylamine/sulfur dioxide copolymer that comprises
a constituent unit (A) having a structure represented by general formula (I-a) or general formula (I-b), or a structure of an acid addition salt thereof: 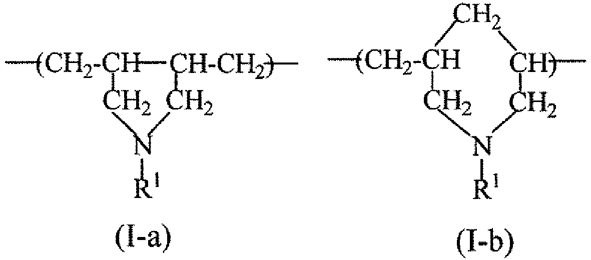
wherein R¹ represents an alkyl group having 1 to 10 carbon atoms, and
a constituent unit (B) having a structure represented by general formula (I-c):

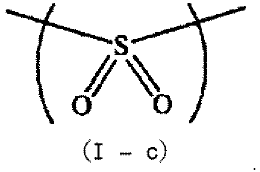

In a method according to the present embodiment, a "sample" is assumed to contain a nucleic acid to be a subject for inhibition of amplification, a "target base sequence" is a base sequence to be a subject for inhibition of amplification, and a "nucleic acid in a sample" is a nucleic acid that may contain a base sequence to be a subject for inhibition of amplification. However, these terms do not mean that a "nucleic acid in a sample" to be subjected to this method actually contains a base sequence to be a subject for inhibition of amplification.

A "clamp nucleic acid" according to the present embodiment has a base sequence complementary to a "target base sequence", and amplification of a nucleic acid having the "target base sequence" is inhibited by the presence of such a "clamp nucleic acid".

According to the present embodiment, in a nucleic acid amplification reaction, a diallylamine/sulfur dioxide copolymer coexists with a clamp nucleic acid having a base sequence complementary to a target base sequence, which enhances the effect of the clamp nucleic acid on inhibiting amplification of a nucleic acid having the target base sequence, and selectively inhibit amplification of the nucleic acid even when a large amount of the nucleic acid having the target base sequence is present in the sample.

Other matters as to a sample and a nucleic acid in the sample are as described in the method for detecting mutation in a target base sequence, and preferred embodiments and specific examples thereof are the same as those of the method. Further, the details of the clamp nucleic acid and the diallylamine/sulfur dioxide copolymer are also as described in the method for detecting mutation in a target base sequence, and preferred embodiments and specific examples thereof are the same as those of the method. Further, the details of implementation of a nucleic acid amplification reaction and reagents for implementing the nucleic acid amplification reaction are also as described in the method and kit for detecting mutation in a target base sequence, and preferred embodiments and specific examples are the same as those of the method and kit. However, in the method of the present embodiment, it is not essential to detect inhibition of amplification of a nucleic acid having a predetermined target base sequence, and steps and reagents necessary for such detection may be implemented or used as necessary.

### 4. Kit for Inhibiting Amplification of Nucleic Acid Having Predetermined Target Base Sequence in Nucleic Acid Amplification Reaction

In still another embodiment, the present invention provides a kit for inhibiting amplification of a nucleic acid having a target base sequence in a nucleic acid amplification reaction comprising:
a clamp nucleic acid having a base sequence complementary to the target base sequence and containing at least one artificial nucleotide residue;
a diallylamine/sulfur dioxide copolymer that comprises
a constituent unit (A) having a structure represented by general formula (I-a) or general formula (I-b), or a structure of an acid addition salt thereof: 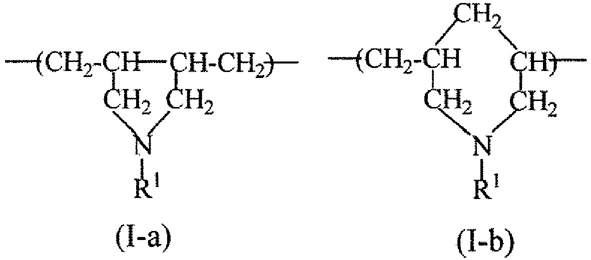
wherein R¹ represents an alkyl group having 1 to 10 carbon atoms, and
a constituent unit (B) having a structure represented by general formula (I-c): 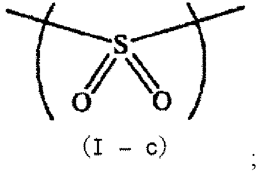
optionally a primer set capable of amplifying a region containing a target base sequence by a nucleic acid amplification method; and
optionally other reagents for implementing a nucleic acid amplification reaction.

The meanings of the terms "sample", "target base sequence", "nucleic acid in a sample", and "clamp nucleic acid" in the present embodiment are the same as those described in the method for inhibiting amplification of a nucleic acid having a target base sequence in a nucleic acid amplification reaction.

Further, other matters as to a sample and a nucleic acid in the sample are as described in the method for detecting mutation in a target base sequence, and preferred embodiments and specific examples thereof are the same as those of the method. Further, the details of the clamp nucleic acid and the diallylamine/sulfur dioxide copolymer are also as described in the method for detecting mutation in a target base sequence, and preferred embodiments and specific examples thereof are the same as those of the method. Further, the details of implementation of a nucleic acid amplification reaction and reagents for implementing the nucleic acid amplification reaction are also as described in the method and kit for detecting mutation in a target base sequence, and preferred embodiments and specific examples are the same as those of the method.

### 5. Use of a Nucleic Acid Amplification Inhibition Enhancing Agent for Enhancing Inhibition of Nucleic Acid Amplification by Clamp Nucleic Acid

In still another embodiment, the present invention provides the use of a nucleic acid amplification inhibition enhancing agent for enhancing a nucleic acid amplification inhibitory effect by a clamp nucleic acid,
the enhancing agent comprising
a diallylamine/sulfur dioxide copolymer comprising:
   a constituent unit (A) having a structure represented by general formula (I-a) or general formula (I-b), or an acid addition salt thereof: 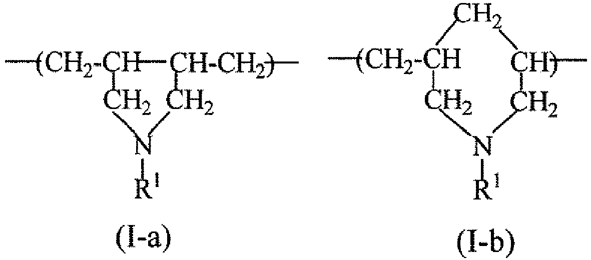
   wherein R¹ represents an alkyl group having 1 to 10 carbon atoms s, and
   a constituent unit (B) having a structure represented by general formula (I-c):

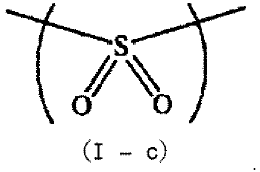

The "nucleic acid amplification" implemented with this enhancing agent is performed with "a clamp nucleic acid having a base sequence complementary to a target base sequence and containing at least one artificial nucleotide residue". The meanings of the terms "sample", "target base sequence", "nucleic acid in a sample", and "clamp nucleic acid" according to the present embodiment are the same as those described in the method for inhibiting amplification of a nucleic acid having a target base sequence in a nucleic acid amplification reaction.

Further, the "nucleic acid amplification inhibition enhancing agent" according to the present embodiment includes the above-described diallylamine/sulfur dioxide copolymer. This is also as described in the method for detecting mutation in a target base sequence, and preferred embodiments and specific examples are the same as those of the method.

### EXAMPLES

Hereinafter, the present invention will be more specifically described by way of Examples, but the present invention is not intended to be limited to these.

### 1. Influence of Addition of Polymer on PCR Reaction

Diallylamine/sulfur dioxide copolymers having a constituent unit (A) and a constituent unit (B) as described above (Examples), or polymers having other constituent units (Comparative Examples) were added to PCR reaction mixtures, and the influence on PCR reactions was verified.

### 1-1. Polymer Aqueous Solution Used

Each of the polymers shown in the following table was dissolved in water, and a polymer aqueous solution having a pH of 7.0 and a concentration of 10% by mass was prepared. The product names in the table are all those of Nittobo Medical Co., Ltd., and the polymerization conditions for polymers 1 to 3 are as follows.

Polymer 1: Into a 100-ml three-neck flask equipped with a stirrer, a thermometer, and a glass stopper, 19.32 g of diluting water (amount to have a monomer concentration of 60% by mass) and 0.25 mol of a diallylethylamine hydrochloride aqueous solution were charged. After 0.25 mol of sulfur dioxide was charged under cooling at 20°C or lower, a 28.5% by mass ammonium persulfate (APS) aqueous solution was added in an amount such that the amount of ammonium persulfate in the aqueous solution was 1.5% by mass with respect to the whole amount of monomers, the amount being added in 10 divided portions, and polymerization was performed at 15 to 30°C.

Polymer 2: Into a 100-ml three-neck flask equipped with a stirrer, a thermometer, and a glass stopper, 4.29 g of diluting water (amount to have a monomer concentration of 70% by mass), 0.13 mol of a diallylpropylamine hydrochloride aqueous solution, and 0.27 g of 35% by mass hydrochloric acid were charged. 0.13 mol of sulfur dioxide was charged under cooling at 20°C or lower, then a 28.5% by mass ammonium persulfate (APS) aqueous solution was added in an amount such that the amount of ammonium persulfate in the aqueous solution was 1.5% by mass with respect to the whole amount of monomers, the amount being added in 10 divided portions, and polymerization was performed at 15 to 30°C.

Polymer 3: Into a 1-L four-neck flask equipped with a stirrer, a thermometer, and a cooling pipe, 248.74 g (1.0 mol) of 65.0% diallyldimethylammonium chloride, 49.03 g (0.5 mol) of maleic anhydride, 89.77 g of distilled water, and 32.03 g (0.5 mol) of sulfur dioxide were charged, and the resulting mixture was cooled to 20°C. A 28.5% by mass ammonium persulfate (APS) aqueous solution was added in an amount such that the amount of ammonium persulfate was 1.5% by mass with respect to the whole amount of monomers, the amount being added in 9 divided portions. The internal temperature was raised to 60°C, the ammonium persulfate (APS) aqueous solution was further added in an amount such that the amount of ammonium persulfate was 5.0% by mass with respect to the whole amount of monomers, the amount being added in 5 divided portions, and the resulting mixture was reacted for 72 hours.

**[Table 1]**

| | Compound name (polymer product name or polymerization conditions) | Constituent unit (A)* | Constituent unit (B) | Constituent unit (A) : constituent unit (B) (molar ratio) | Weight average molecular weight (Mw) |
|---|---|---|---|---|---|
| Example 1 | Diallylmethylamine hydrochloride/sulfur dioxide copolymer | Diallylmethylamine hydrochloride | Sulfur dioxide | 1:1 | 3000 |
| Example 2 | Diallylethylamine hydrochloride/sulfur dioxide copolymer (polymer 1) | Diallylethylamine hydrochloride | Sulfur dioxide | 1:1 | 3000 |
| Example 3 | Diallylpropylamine hydrochloride/sulfur dioxide copolymer (polymer 2) | Diallylpropylamine hydrochloride | Sulfur dioxide | 1:1 | 3000 |
| Comparative Example 1 | Diallylamine hydrochloride /sulfur dioxide copolymer | Diallylamine hydrochloride | Sulfur dioxide | 1:1 | 5000 |
| Comparative Example 2 | Diallyldimethylammonium chloride/sulfur dioxide copolymer | Diallyldimethylammonium chloride | Sulfur dioxide | 1:1 | 4000 |
| Comparative Example 3 | Diallyldimethylammonium chloride/sulfur dioxide/ maleic acid copolymer (polymer 3) | - | - | - | 20000 |

| | | | | | |
|---|---|---|---|---|---|
| *) In Comparative Examples 1 to 3, the diallylamine unit does not correspond to the constituent unit (A), but is described in the column of Constituent unit (A) in the present table for the convenience of description. Further, the polymer of Comparative Example 3 is composed of three constituent units of diallyldimethylammonium chloride, sulfur dioxide, and maleic acid, and the molar ratio of each constituent unit is 2 : 1 : 1. | | | | | |

### 1-2. PCR Reaction Mixture

A PCR reaction mixture having the composition shown in the following table was prepared.

**[Table 2]**

| Component | Content |
|---|---|
| 2×PCR Buffer TB Green Premix Dimer Eraser*¹ (manufactured by Takara Bio, Inc.) | 10.0µL |
| 10 µM Forward primer (synthesis entrusted to Fasmac Co., Ltd.) | 0.6µL |
| 10 µM Reverse primer (synthesis entrusted to Fasmac Co., Ltd.) | 0.6µL |
| 50×ROX dye (manufactured by Takara Bio, Inc.) | 0.4µL |
| 50 ng/µL template DNA | 1.0µL |
| Polymer aqueous solution | 2.0µL |
| Nuclease-free water (manufactured by Thermo Fisher Scientific, Inc.) | 5.4µL |

| | |
|---|---|
| ^{*1} contains Taq polymerase (TaKaRa Ex Taq HS), dNTP Mixture, Mg²⁺, and a fluorescent dye (TB Green). The final concentration of each polymer aqueous solution in the PCR reaction mixture was 0.01% by mass. | |

### 1-3. Primers and Template DNA

The sequences of a forward primer and a reverse primer used in the PCR reaction are as shown in the following table.

**[Table 3]**

| | |
|---|---|
| Forward primer | GCCTGCTGAAAATGACTGAATATA (SEQ ID NO:1) |
| Reverse primer | CAAGATTTACCTCTATTGTTGGA (SEQ ID NO:2) |

Each primer was designed using Primer3 (obtained from http://frodo.wi.mit.edu) to target the KRAS gene (the base sequence of the gene can be found at, for example, NCBI (DB name)).

As a template DNA, a genome extracted from HCC70 cells containing the following base sequence (sequence to be amplified) was used.

GCCTGCTGAAAATGACTGAATATAAACTTGTGGTAGTTGGAGCTGGTGGCGTAGGCAAGAGTGCCTTGACGATAC
AGCTAATTCAGAATCATTTTGTGGACGAATATGATCCAACAATAGAGGTAAATCTTG (SEQ ID NO:3)

The above-described base sequence, and a base sequence adjacent thereto in the genome of HCC70 cells are as follows.

TGAGTTTGTATTAAAAGGTACTGGTGGAGTATTTGATAGTGTATTAACCTTATGTGTGACATGTTCTAATATAGT
CACATTTTCATTATTTTTATTATAAGGCCTGCTGAAAATGACTGAATATAAACTTGTGGTAGTTGGAGCTGGTGG
CGTAGGCAAGAGTGCCTTGACGATACAGCTAATTCAGAATCATTTTGTGGACGAATATGATCCAACAATAGAGGT
AAATCTTGTTTTAATATGCATATTACTGGTGCAGGACCATTCTTTGATACACGATAAAGGTTTCTCTGACCATTT
(SEQ ID NO:4)

### 1-4. Real-Time PCR System

Step One Plus real-time PCR system (manufactured by Thermo Fisher Scientific, Inc.) was used.

### 1-5. PCR Reaction

20 µL of the prepared PCR reaction mixture was loaded in a real-time PCR system, and after performing step (i) described in the following table, 40 cycles of steps (ii) to (iv) were repeated.

**[Table 4]**

| Step | Temperature | Time |
|---|---|---|
| i | 95°C | 30 seconds |
| ii | 95°C | 5 seconds |
| iii | 55°C | 30 seconds |
| iv | 72°C | 30 seconds |
| v | 95°C | 15 seconds |
| vi | 60°C | 60 seconds |
| vii | 95°C | 15 seconds |

After completion of 40 cycles, steps (v) to (vii) were performed, and the reaction was terminated. The amount of amplificated DNA was monitored by measuring the fluorescence intensity of the reaction mixture for each cycle. Further, after completion of the reaction, whether the PCR reaction was inhibited was determined based on whether the fluorescence intensity reached the Threshold Line. The Threshold Line was automatically calculated by the above-described apparatus. In the case where the Threshold Line was unavoidably set to too low in some experiments, the ΔRn value was manually set in the range between 0.7 and 1.2 in order to ensure a comparison with other experiments on the same level.

### 1-5. Results

The success or failure of the PCR reaction is shown in the following table.

**[Table 5]**

| | Polymer | Success or failure of PCR reaction (○/×) |
|---|---|---|
| Example 1 | Diallylmethylamine hydrochloride/sulfur dioxide copolymer | ○ |
| Example 2 | Diallylethylamine hydrochloride/sulfur dioxide copolymer | ○ |
| Example 3 | Diallylpropylamine hydrochloride/sulfur dioxide copolymer | ○ |
| Comparative Example 1 | Diallylamine hydrochloride/sulfur dioxide copolymer | × |
| Comparative Example 2 | Diallyldimethylammonium chloride/sulfur dioxide copolymer | × |
| Comparative Example 3 | Diallyldimethylammonium chloride/sulfur dioxide/maleic acid copolymer | × |

| | | |
|---|---|---|
| O: Did not inhibit PCR reaction. ×: Inhibited PCR reaction. | | |

FIG. 1 shows, as an example of a nucleic acid amplification curve in a case where the PCR reaction is not inhibited, a nucleic acid amplification curve in a case where the polymer of Example 1 (diallylmethylamine hydrochloride/sulfur dioxide copolymer) is added, and as an example of a nucleic acid amplification curve in a case where the PCR reaction is inhibited, a nucleic acid amplification curve in a case where Comparative Example 1 (diallylamine hydrochloride/sulfur dioxide copolymer) is added.

As described above, when the polymers of Examples 1 to 3, which are diallylamine/sulfur dioxide copolymers containing a cationic constituent unit containing a tertiary amine in the structure, and a constituent unit derived from a sulfur dioxide monomer, were added, the PCR reaction was not inhibited. On the other hand, when the polymers of Comparative Examples 1 to 3, which are copolymers containing a cationic constituent unit containing a secondary amine or a quaternary amine, and a constituent unit derived from a sulfur dioxide monomer or sulfur dioxide and maleic acid, were added, the PCR reaction was inhibited.

### 2. Polymer- or Monomer-Induced Enhancement of Effect of Selectively Inhibiting Amplification of Wild-Type Gene by BNA Clamp

The polymers of Examples 1 to 3 that were verified not to inhibit the PCR reaction in Test 1, and a monomer (diallylmethylamine hydrochloride) from which a constituent unit of the polymers was derived, were evaluated for enhancement of the effect of inhibiting amplification of wild-type KRAS gene in BNA clamp PCR.

### 2-1. Polymers or Monomer Used

In the present test, the polymers of Examples 1 to 3 and diallylmethylamine hydrochloride (Comparative Example 4) were used. Each polymer or monomer was dissolved in water, and a polymer aqueous solution or a monomer aqueous solution having a pH of 7.0 and a concentration of 0.25% by mass was prepared.

### 2-2. Kit Used

Appendages of BNA Clamp KRAS Enrichment Kit (Riken Genesis Co., Ltd.), which is a kit for detecting a mutant KRAS gene, were used. The details will be described below, but the kit appendages were used for a primer set and a BNA clamp. The reaction conditions for real-time PCR were in accordance with the kit protocol.

### 2-3. Primers, BNA Clamp, and Template DNA

The forward primer, reverse primer and BNA clamp shown in the following table were used.

**[Table 6]**

| | |
|---|---|
| Forward primer | CTGAATATAAACTTGTGGTAGTTGG (SEQ ID NO:5) |
| Reverse primer | GTCCTGCACCAGTAATATGC (SEQ ID NO:6) |
| BNA clamp | C*C*T*AC*GC*C*AC*C* (SEQ ID NO:7) |

| | |
|---|---|
| *: Represents the base of an artificial nucleotide. | |

Further, as a template DNA having a wild-type KRAS gene containing a standard base sequence, a genome extracted from HCC70 cells containing the following base sequence (sequence to be amplified; bases different from those of the base sequence of SEQ ID NO: 10 that will be described below are underlined and bolded) was used.

CTGAATATAAACTTGTGGTAGTTGGAGCTGGTGGCGTAGGCAAGAGTGCCTTGACGATACAGCTAATTCAGAATC
ATTTTGTGGACGAATATGATCCAACAATAGAGGTAAATCTTGTTTTAATATGCATATTACTGGTGCAGGAC
(SEQ ID NO:8)

In the genome of HCC70 cells, the above-described base sequence and a base sequence adjacent thereto are as follows (bases different from those of the base sequence of SEQ ID NO:11 that will be described later are underlined and bolded).

TGAGTTTGTATTAAAAGGTACTGGTGGAGTATTTGATAGTGTATTAACCTTATGTGTGACATGTTCTAATATAGT
CACATTTTCATTATTTTTATTATAAGGCCTGCTGAAAATGACTGAATATAAACTTGTGGTAGTTGGAGCTGGTGG
CGTAGGCAAGAGTGCCTTGACGATACAGCTAATTCAGAATCATTTTGTGGACGAATATGATCCAACAATAGAGGT
AAATCTTGTTTTAATATGCATATTACTGGTGCAGGACCATTCTTTGATACACGATAAAGGTTTCTCTGACCATTT
(SEQ ID NO:9)

On the other hand, as a template DNA having a mutation relative to a standard base sequence , a genome of MDA-MB-231 cells having a mutant KRAS gene, which contains the following base sequence (sequence to be amplified; bases different from those of the base sequence of SEQ ID NO:8 described above are underlined and bolded), was used.

CTGAATATAAACTTGTGGTAGTTGGAGCTGGTGACGTAGGCAAGAGTGCCTTGACGATACAGCTAATTCAGAATC
ATTTTGTGGACGAATATGATCCAACAATAGAGGTAAATCTTGTTTTAATATGCATATTACTGGTGCAGGAC
(SEQ ID NO:10)

In the genome of MDA-MB-231 cell, the above-described base sequence and a base sequence adjacent thereto are as follows (bases different from those of the base sequence of SEQ ID NO:9 are underlined and bolded).

TGAGTTTGTATTAAAAGGTACTGGTGGAGTATTTGATAGTGTATTAACCTTATGTGTGACATGTTCTAATATAGT
CACATTTTCATTATTTTTATTATAAGGCCTGCTGAAAATGACTGAATATAAACTTGTGGTAGTTGGAGCTGGTGA
CGTAGGCAAGAGTGCCTTGACGATACAGCTAATTCAGAATCATTTTGTGGACGAATATGATCCAACAATAGAGGT
AAATCTTGTTTTAATATGCATATTACTGGTGCAGGACCATTCTTTGATACACGATAAAGGTTTCTCTGACCATTT
(SEQ ID NO:11)

### 2-4. BNA Clamp PCR Reaction Mixture

Reaction mixtures 1 to 4 having the compositions shown in the following table were prepared. In the reaction mixture 1 and the reaction mixture 2, the concentration of the polymer or the monomer was 0.025% by mass.

**[Table 7]**

| Reaction mixture 1 (polymer or monomer +, BNA clamp +) | |
|---|---|
| Component | Content |
| PCR Buffer TB Green Premix Dimer Eraser*¹ (manufactured by Takara Bio, Inc.) | 10.0µL |
| 10 µM Forward primer (manufactured by Riken Genesis Co., Ltd.) | 0.6µL |
| 10 µM Reverse primer (manufactured by Riken Genesis Co., Ltd.) | 0.6µL |
| ROX dye (manufactured by Takara Bio, Inc.) | 0.4µL |
| 10 µM BNA clamp (manufactured by Riken Genesis Co., Ltd.) | 0.2µL |
| Template DNA (50 ng/µL genomic DNA of HCC70 cells, or 50 ng/µL genomic DNA of MDA-MB-231 cells) | 1.0µL |
| 0.25% by mass polymer aqueous solution or monomer aqueous solution | 2.0µL |
| Nuclease-free water (manufactured by Thermo Fisher Scientific, Inc.) | 5.2µL |

| | |
|---|---|
| ^{*1} contains Taq polymerase (TaKaRa Ex Taq HS), dNTP Mixture, Mg²⁺, and a fluorescent dye (TB Green). | |

**[Table 8]**

| Reaction mixture 2 (polymer or monomer +, BNA clamp -) | |
|---|---|
| Component | Content |
| PCR Buffer TB Green Premix Dimer Eraser^{*1} (manufactured by Takara Bio, Inc.) | 10.0µL |
| 10 µM Forward primer (manufactured by Riken Genesis Co., Ltd.) | 0.6µL |
| 10 µM Reverse primer (manufactured by Riken Genesis Co., Ltd.) | 0.6µL |
| ROX dye (manufactured by Takara Bio, Inc.) | 0.4µL |
| 10 µM BNA clamp (manufactured by Riken Genesis Co., Ltd.) | 0µL |
| Template DNA (50 ng/µL genomic DNA of HCC70 cells, or 50 ng/µL genomic DNA of MDA-MB-231 cells) | 1.0µL |
| 0.25% by mass polymer aqueous solution or monomer aqueous solution | 2.0µL |
| Nuclease-free water (manufactured by Thermo Fisher Scientific, Inc.) | 5.4µL |

| | |
|---|---|
| ^{*1} contains Taq polymerase (TaKaRa Ex Taq HS), dNTP Mixture, Mg²⁺, and a fluorescent dye (TB Green). | |

**[Table 9]**

| Reaction mixture 3 (polymer or monomer -, BNA clamp +) | |
|---|---|
| Component | Content |
| PCR Buffer TB Green Premix Dimer Fraser^{*1} (manufactured by Takara Bio, Inc.) | 10.0µL |
| 10 µM Forward primer (manufactured by Riken Genesis Co., Ltd.) | 0.6µL |
| 10 µM Reverse primer (manufactured by Riken Genesis Co., Ltd.) | 0.6µL |
| ROX dye (manufactured by Takara Bio, Inc.) | 0.4µL |
| 10 µM BNA clamp (manufactured by Riken Genesis Co., Ltd.) | 0.2µL |
| Template DNA (50 ng/µL genomic DNA of HCC70 cells, or 50 ng/µL genomic DNA of MDA-MB-231 cells) | 1.0µL |
| 0.25% by mass polymer aqueous solution or monomer aqueous solution | 0µL |
| Nuclease-free water (manufactured by Thermo Fisher Scientific, Inc.) | 7.2µL |

| | |
|---|---|
| ^{*1} contains Taq polymerase (TaKaRa Ex Taq HS), dNTP Mixture, Mg²⁺, and a fluorescent dye (TB Green). | |

**[Table 10]**

| Reaction mixture 4 (polymer or monomer -, BNA clamp -) | |
|---|---|
| Component | Content |
| PCR Buffer TB Green Premix Dimer Eraser*¹ (manufactured by Takara Bio, Inc.) | 10.0µL |
| 10 µM Forward primer (manufactured by Riken Genesis Co., Ltd.) | 0.6µL |
| 10 µM Reverse primer (manufactured by Riken Genesis Co., Ltd.) | 0.6µL |
| ROX dye (manufactured by Takara Bio, Inc.) | 0.4µL |
| 10 µM BNA clamp (manufactured by Riken Genesis Co., Ltd.) | 0µL |
| Template DNA (50 ng/µL genomic DNA of HCC70 cells, or 50 ng/µL genomic DNA of MDA-MB-231 cells) | 1.0µL |
| 0.25% by mass polymer aqueous solution or monomer aqueous solution | 0µL |
| Nuclease-free water (manufactured by Thermo Fisher Scientific, Inc.) | 7.4µL |

| | |
|---|---|
| ^{*1} contains Taq polymerase (TaKaRa Ex Taq HS), dNTP Mixture, Mg²⁺, and a fluorescent dye (TB Green). | |

### 2-5. Real-Time PCR System

Step One Plus real-time PCR system (Thermo Fisher Scientific, Inc.) was used.

### 2-6. PCR Reaction

20 µL of each of the prepared reaction mixtures was placed in a PCR tube, the PCR tube was set in a real-time PCR system, step (i) described in the following table was performed, and then 50 cycles of steps (ii) to (iv) were repeated. After completion of 50 cycles, steps (v) to (vii) were performed, and the reaction was terminated. The amount of amplified DNA was monitored by measuring the fluorescence intensity of the reaction mixture for each cycle to obtain an amplification curve of each reaction mixture.

**[Table 11]**

| Step | Temperature | Time |
|---|---|---|
| i | 95°C; | 30 seconds |
| ii | 95°C | 5 seconds |
| iii | 58°C | 30 seconds |
| iv | 72°C | 30 seconds |
| v | 95°C | 15 seconds |
| vi | 60°C | 60 seconds |
| vii | 95°C | 15 seconds |

### 2-7. Calculation of ΔCt (WT) and ΔCt (Mutant) with or without addition of polymer, calculation of ΔΔCt with or without addition of polymer, and calculation of ΔΔΔCt

Reaction mixture 1 (with addition of polymer, with addition of BNA clamp), reaction mixture 2 (with addition of polymer, without addition of BNA clamp), reaction mixture 3 (without addition of polymer, with addition of BNA clamp), and reaction mixture 4 (without addition of polymer, without addition of BNA clamp) were subjected to the above-described PCR reaction using a wild-type KRAS gene or a mutant KRAS gene as a template DNA, to obtain amplification curves, and in the amplification curve under each condition, each Ct value was obtained from the set Threshold Line (in most experimental sections, the Threshold Line was automatically calculated by the apparatus). In the case where the Threshold Line was unavoidably set to too low in some experiments, the ΔRn value was manually set in the range between 0.7 and 1.2 in order to ensure a comparison with other experiments on the same level.

Next, ΔCt (WT) and ΔCt (Mutant) in the case of not adding a polymer were calculated by the following formulas.
ΔCt (WT) in case of no addition of polymer = Ct (WT) in case of reaction mixture 3 (without addition of polymer, with addition of BNA clamp) - Ct (WT) in case of reaction mixture 4 (without addition of polymer, without addition of BNA clamp)
ΔCt (Mutant) in case of no addition of polymer = Ct (Mutant) in case of reaction mixture 3 (without addition of polymer, with addition of BNA clamp) - Ct (Mutant) in case of reaction mixture 4 (without addition of polymer, without addition of BNA clamp)

Further, in order to verify that there is no non-specific amplification in the intercalator method, ΔΔCt in the case of no addition of polymer was calculated by the following formula.
ΔΔCt in case of no addition of polymer = ΔCt (WT) in case of no addition of polymer - ΔCt (Mutant) in case of no addition of polymer

It is understood that there is no non-specific amplification in the intercalator method when ΔΔCt > 0 in the case of no addition of polymer.

Next, ΔCt (WT) and ΔCt (Mutant) in the case of addition of polymer were calculated by the following formulas, and further, ΔΔCt in the case of addition of polymer was calculated in order to verify whether there is no non-specific amplification in the intercalator method.
ΔCt (WT) in case of addition of polymer = Ct (WT) in case of reaction mixture 1 (with addition of polymer, with addition of BNA clamp) - Ct (WT) in case of reaction mixture 2 (with addition of polymer, without addition of BNA clamp)
ΔCt (Mutant) in case of addition of polymer = Ct (Mutant) in case of reaction mixture 1 (with addition of polymer, with addition of BNA clamp) - Ct (Mutant) in case of reaction mixture 2 (with addition of polymer, without addition of BNA clamp)
ΔΔCt in case of addition of polymer = ΔCt (WT) in case of addition of polymer - ΔCt (Mutant) in case of addition of polymer

It is understood that when ΔΔCt > 0 in the case of addition of polymer, there is no non-specific amplification in the intercalator method.

Finally, as an index for evaluating the polymers as to enhancement of the effect of selectively inhibiting amplification of a wild-type gene by the BNA clamp, ΔΔΔCt was calculated by the following formula.
ΔΔΔCt = ΔΔCt in case of addition of polymer - ΔΔCt in case of no addition of polymer

Even taking into account possible fluctuations of results in the test, when ΔΔΔCt≥1.00 in the case of using a mutation detection method based on the number of amplification cycles of a wild-type gene, such as the intercalator method, it can be evaluated that the effect of selectively inhibiting amplification of the wild-type gene by a BNA clamp was sufficiently enhanced. Further, when ΔΔΔCt ≥ 4.00 in the case of using a mutation detection method based on the number of amplification cycles of a wild-type gene, such as an intercalator method, it can be evaluated that the effect of selectively inhibiting amplification of the wild-type gene by the BNA clamp was remarkably enhanced. In addition, when ΔΔΔCt ≥ 8.00 in the case of using a mutation detection method based on the number of amplification cycles of a wild-type gene, such as an intercalator method, it can be evaluated that the effect of selectively inhibiting amplification of the wild-type gene by the BNA clamp was extremely remarkably enhanced. ΔΔΔCt = 3.20 is an indication that the sensitivity is improved by about 10 times.

### 2-8. Results

The test results are summarized below.

**[Table 12]**

| | Polymer or monomer | ΔΔΔCt |
|---|---|---|
| Example 1 | Diallylmethylamine hydrochloride/sulfur dioxide copolymer | 11.74 |
| Example 2 | Diallylethylamine hydrochloride/sulfur dioxide copolymer | 6.05 |
| Example 3 | Diallylpropylamine hydrochloride/sulfur dioxide copolymer | 2.91 |
| Comparative Example 4 | Diallylmethylamine hydrochloride | 0.95 |

From the above-described test results, it became clear that when the polymers of Examples 1 to 3 were added, the effect of selectively inhibiting amplification of the wild-type gene by the BNA clamp was enhanced without inhibiting the amplification of the mutant gene.

On the other hand, the monomer of Comparative Example 4 also enhanced the effect of selectively inhibiting amplification of the wild-type gene by the BNA clamp, but the enhancement of inhibition was not as sufficient as with the polymers in Examples 1-3.

In addition, when the monomer was added, an error (variation in results) was large and the effect was not stable. This error (variation in results) may be due to the radical polymerization of the monomer, since the monomer can generate radicals by heat, light, or other means.

From this test result, polymer structures were more advantageous than a monomer from the viewpoint that the effect of selectively inhibiting amplification of the wild-type gene by the BNA clamp can be more greatly enhanced with good reproducibility.

### 3. Sensitivity Enhancement of BNA Clamp PCR by Addition of Polymer

An attempt was made to increase the sensitivity of BNA clamp PCR with the polymer of Example 1, by which enhancement of the effect of selectively inhibiting amplification of the wild-type gene by BNA clamp was verified in the above-described test.

### 3-1. Polymer Used

The polymer of Example 1 was dissolved in water to prepare a polymer aqueous solution having a pH of 7.0 and a concentration of 10% by mass. The obtained 10% by mass polymer aqueous solution was further diluted with water to prepare a 0.1% by mass polymer aqueous solution, which was used for the preparation of a reaction mixture.

### 3-2. Kit Used

The appendages of the same kit as that in Test 2 were used.

### 3-3. Template DNA

As a template DNA having a standard base sequence, used was a genome of HCC70 cells having a wild-type KRAS gene containing the base sequence of SEQ ID NO:8. Further, as a template DNA having a mutation relative to the standard base sequence, used was a genome of MDA-MB-231 cells having a mutant KRAS gene containing the base sequence of SEQ ID NO:10.

In this test, the template DNA of the wild-type gene and the template DNA of the mutant gene were mixed such that the % by mass of the template DNA of the mutant gene in the whole template DNAs was as shown in the following table while the whole amount of the template DNAs was adjusted to 50 ng in 2.0 µL, and each of the obtained template DNA mixtures and the template DNA of the wild-type gene were used in the PCR reaction.

**[Table 13]**

| Mutant ratio | Number of Mutant copies |
|---|---|
| 10% Mutant(5ng) | 1.65×10³ copies |
| 1% Mutant(0.5ng) | 1.65×10² copies |
| 0.1% Mutant(0.05ng) | 1.65×10 copies |
| 0.01% Mutant(0.005ng) | 1.65 copies |

### 3-4. Primers and BNA Clamp

The same primers and BNA clamp as those used in Test 2 were used.

### 3-5. BNA Clamp PCR Reaction Mixture

Reaction mixtures 1 and 2 with the compositions as shown in the following table were prepared. In the reaction mixture 1, the polymer concentration was 0.01% by mass.

**[Table 14]**

| Reaction mixture 1 (polymer +, BNA clamp +) | |
|---|---|
| Component | Content |
| PCR Buffer TB Green Premix Dimer Eraser^{*1} (manufactured by Takara Bio, Inc.) | 10.0µL |
| 10 µM Forward primer (manufactured by Riken Genesis Co., Ltd.) | 0.6µL |
| 10 µM Reverse primer (manufactured by Riken Genesis Co., Ltd.) | 0.6µL |
| ROX dye (manufactured by Takara Bio, Inc.) | 0.4µL |
| 10 µM BNA clamp (manufactured by Riken Genesis Co., Ltd.) | 0.2µL |
| 50 ng of template DNA (WT^{*2} or mixture^{*4} of WT and Mutant^{*3}) | 2.0µL |
| 0.1% by mass polymer aqueous solution | 2.0µL |
| Nuclease-free water (manufactured by Thermo Fisher Scientific, Inc.) | 4.2µL |

| | |
|---|---|
| ^{*1} contains Taq polymerase (TaKaRa Ex Taq HS), dNTP Mixture, Mg²⁻, and a fluorescent dye (TB Green). ^{*2} Genomic DNA of HCC70 cells ^{*3} Genomic DNA of MDA-MB-231 cells ^{*4} contains 50 ng of the template DNA in 2.0 µL of whole mixture. | |

**[Table 15]**

| Reaction mixture 2 (polymer -, BNA clamp +) | |
|---|---|
| Component | Content |
| PCR Buffer TB Green Premix Dimer Eraser*¹ (manufactured by Takara Bio, Inc.) | 10.0µL |
| 10 µM Forward primer (manufactured by Riken Genesis Co., Ltd.) | 0.6µL |
| 10 µM Reverse primer (manufactured by Riken Genesis Co., Ltd.) | 0.6µL |
| ROX dye (manufactured by Takara Bio, Inc.) | 0.4µL |
| 10 µM BNA clamp (manufactured by Riken Genesis Co., Ltd.) | 0.2µL |
| 50 ng of template DNA (WT^{*2} or mixture^{*4} of WT and Mutant^{*3}) | 2.0µL |
| Polymer aqueous solution | 0µL |
| Nuclease-free water (manufactured by Thermo Fisher Scientific, Inc.) | 6.2µ^{*2} |

| | |
|---|---|
| ^{*1} contains Taq polymerase (TaKaRa Ex Taq HS), dNTP Mixture, Mg²⁻, and a fluorescent dye (TB Green). ^{*2} Genomic DNA of HCC70 cells ^{*3} Genomic DNA of MDA-MB-231 cells ^{*4} contains 50 ng of the template DNA in 2.0 µL of whole mixture. | |

### 3-6. Real-Time PCR System and PCR Reaction

PCR reactions were carried out under the same conditions as those in Test 2, using the same real-time PCR system as that in Test 2.

### 3-7. Determination of δCt (Mutant 10% to 0.01%) with or without Addition of Polymer and Calculation of δδCt (Mutant 10% to 0.01%) with or without Addition of Polymer

The reaction mixture 1 (with addition of polymer, with addition of BNA clamp) and the reaction mixture 2 (without addition of polymer, with addition of BNA clamp) were subjected to the above-described PCR reaction with the template DNA or each template DNA mixture to obtain amplification curves, Ct (Mutant 10% to 0%) was determined in the same manner as in Test 2 from the amplification curve under each condition, and as shown in the following table, each δCt (Mutant 10% to 0.01%) with or without addition of polymer was calculated by subtracting Ct (Mutant 10% to 0.01%) at each mutant gene template concentration from Ct (Mutant 0%, WT).

**[Table 16]**

| Calculation formula of δCt (Mutant 10% to 0.01%) in case of no addition of polymer | | | | | |
|---|---|---|---|---|---|
| Mutant | 10% | 1% | 0.10% | 0.01% | 0% |
| Ct | ① | ② | ③ | ④ | ⑤ |
| δCt | ⑤-① | ⑤-② | ⑤-③ | ⑤-④ | |

**[Table 17]**

| Calculation formula of δCt (Mutant 10% to 0.01%) in case of addition of polymer | | | | | |
|---|---|---|---|---|---|
| Mutant | 10% | 1% | 0.10% | 0.01% | 0% |
| Ct | A | B | C | D | E |
| δCt | E-A | E-B | E-C | E-D | |

Finally, each δδCt (Mutant 10% to 0.01%) was calculated by subtracting δCt (Mutant 10% to 0.01%) in the case of no addition of polymer from δCt (Mutant 10% to 0.01%), in the case of addition of polymer.

When δδCt > 0, it means that a mutant gene is specifically detected by adding a polymer, and the amplification curve of the mutant gene is distinguished from the amplification curve of the wild-type gene (Please refer to Patent Literature 11).

### 3-8. Results

The test results are summarized in the following table and FIG. 2.

**[Table 18]**

| Mutant ratio | δCt in case of no addition of polymer | δCt in case of addition of polymer | δδCt |
|---|---|---|---|
| 10% Mutant | 7.88 | 10.68 | 2.80 |
| 1% Mutant | 4.85 | 7.00 | 2.15 |
| 0.1% Mutant | 2.67 | 4.62 | 1.95 |
| 0.01% Mutant | -0.68 | 3.81 | 4.49 |

As a result of this test, it became clear that in the case of no addition of polymer, when the Mutant ratio was 0.01%, δCt was less than 0, and the amplification curve of the mutant gene could not be distinguished from the amplification curve of the wild-type gene; however, in the case where the polymer of Example 1 was added, the amplification curve of the mutant gene could be distinguished from the amplification curve of the wild-type gene even at a Mutant ratio of 0.01%. Although a reaction mixture without addition of BNA clamp was not tested in this experiment, it is clear from the results of Test 2 that there is no non-specific amplification even in a reaction mixture without addition of BNA clamp, regardless of the presence or absence of the addition of polymer.

### 4. Adaptability to Mutant gene Other Than KRAS 1 (Polymer-Induced Enhancement of Effect of Selectively Inhibiting Amplification of Wild-Type Gene by BNA Clamp)

### 4-1. Polymer Used

The polymer of Example 1 was dissolved in water, and a 0.1% by mass polymer solution was prepared, which was used for the preparation of a reaction mixture.

### 4-2. Kit Used

BNA Clamp BRAF Enrichment Kit (Riken Genesis Co., Ltd.), which is a kit for detecting a mutant BRAF gene, was used. For the primer set and BNA clamp, kit appendages were used. The reaction conditions for real-time PCR were in accordance with the kit protocol.

### 4-3. Template DNA

As a template DNA with a standard base sequence, used was a genome of HCC70 cells having a wild-type BRAF gene containing the following base sequence (bases different from those of the base sequence of SEQ ID NO:13 that will be described below are underlined and bolded).

ATAGAAATTAGATCTCTTACCTAAACTCTTCATAATGCTTGCTCTGATAGGAAAATGAGATCTACTGTTTTCCTTTA
CTTACTACACCTCAGATATATTTCTTCATGAAGACCTCACAGTAAAAATAGGTGATTTTGGTCTAGCTACAGTGAAA
TCTCGATGGAGTGGGTCCCATCAGTTTGAACAGTTGTCTGGATCCATTTTGTGGATGGTAAGAATTGAGGCTATTTT
TCCACTGATTAAATTTTTGGCCCTGAGATGCTGCTGAGTTACTAGAAAGTCATTGAAGGTCTCAACTAT (SEQ ID
NO:12)

It should be noted that the above-described sequence is understood to contain a base sequence other than the sequence to be amplified.

On the other hand, as a template DNA having a mutation relative to the standard base sequence, used was a genome of DU4475 cells having a mutant BRAF gene containing the following base sequence (bases different from those of the base sequence of SEQ ID NO: 12 described above are underlined and bolded).

ATAGAAATTAGATCTCTTACCTAAACTCTTCATAATGCTTGCTCTGATAGGAAAATGAGATCTACTGTTTTCCTT
TACTTACTACACCTCAGATATATTTCTTCATGAAGACCTCACAGTAAAAATAGGTGATTTTGGTCTAGCTACAGA
GAAATCTCGATGGAGTGGGTCCCATCAGTTTGAACAGTTGTCTGGATCCATTTTGTGGATGGTAAGAATTGAGGC
TATTTTTCCACTGATTAAATTTTTGGCCCTGAGATGCTGCTGAGTTACTAGAAAGTCATTGAAGGTCTCAACTAT
(SEQ ID NO:13)

It should be noted that the above-described sequence is understood to contain a base sequence other than the sequence to be amplified.

### 4-4. BNA Clamp PCR Reaction Mixture

Reaction mixtures 1 to 4 with the compositions shown in the following table were prepared. The polymer concentrations in the reaction mixtures 1 and 2 were 0.01% by mass.

**[Table 19]**

| Reaction mixture 1 (polymer +, BNA clamp +) | |
|---|---|
| Component | Content |
| PCR Buffer TB Green Premix Dimer Eraser^{*1} (manufactured by Takara Bio, Inc.) | 10.0µL |
| 10 µM Forward primer (manufactured by Riken Genesis Co., Ltd.) | 0.6µL |
| 10 µM Reverse primer (manufactured by Riken Genesis Co., Ltd.) | 0.6µL |
| ROX dye (manufactured by Takara Bio, Inc.) | 0.4µL |
| 10 µM BNA clamp (manufactured by Riken Genesis Co., Ltd.) | 0.2µL |
| Template DNA (50 ng/µL genomic DNA of HCC70 cells, or 50 ng/µL genomic DNA of DU4475 cells) | 1.0µL |
| 0.1% by mass polymer aqueous solution | 2.0µL |
| Nuclease-free water (manufactured by Thermo Fisher Scientific, Inc.) | 5.2µL |

| | |
|---|---|
| ^{*1} contains Taq polymerase (TaKaRa Ex Taq HS), dNTP Mixture, Mg²⁺, and a fluorescent dye (TB Green). | |

**[Table 20]**

| Reaction mixture 2 (polymer +, BNA clamp -) | |
|---|---|
| Component | Content |
| PCR Buffer TB Green Premix Dimer Eraser^{*1} (manufactured by Takara Bio, Inc.) | 10.0µL |
| 10 µM Forward primer (manufactured by Riken Genesis Co., Ltd.) | 0.6µL |
| 10 µM Reverse primer (manufactured by Riken Genesis Co., Ltd.) | 0.6µL |
| ROX dye (manufactured by Takara Bio, Inc.) | 0.4µL |
| 10 µM BNA clamp (manufactured by Riken Genesis Co., Ltd.) | 0µL |
| Template DNA (50 ng/µL genomic DNA of HCC70 cells, or 50 ng/µL genomic DNA of DU4475 cells) | 1.0µL |
| 0.1% by mass polymer aqueous solution | 2.0µL |
| Nuclease-free water (manufactured by Thermo Fisher Scientific, Inc.) | 5.4µL |

| | |
|---|---|
| ^{*1} contains Taq polymerase (TaKaRa Ex Taq HS), dNTP Mixture, Mg²⁺, and a fluorescent dye (TB Green). | |

**[Table 21]**

| Reaction mixture 3 (polymer -, BNA clamp +) | |
|---|---|
| Component | Content |
| PCR Buffer TB Green Premix Dimer Eraser^{*1} (manufactured by Takara Bio, Inc.) | 10.0µL |
| 10 µM Forward primer (manufactured by Riken Genesis Co., Ltd.) | 0.6µL |
| 10 µM Reverse primer (manufactured by Riken Genesis Co., Ltd.) | 0.6µL |
| ROX dye (manufactured by Takara Bio, Inc.) | 0.4µL |
| 10 µM BNA clamp (manufactured by Riken Genesis Co., Ltd.) | 0.2µL |
| Template DNA (50 ng/µL genomic DNA of HCC70 cells, or 50 ng/µL genomic DNA of DU4475 cells) | 1.0µL |
| Polymer aqueous solution | 0µL |
| Nuclease-free water (manufactured by Thermo Fisher Scientific, Inc.) | 7.2µL |

| | |
|---|---|
| ^{*1} contains Taq polymerase (TaKaRa Ex Taq HS), dNTP Mixture, Mg²⁺, and a fluorescent dye (TB Green). | |

**[Table 22]**

| Reaction mixture 4 (polymer -, BNA clamp -) | |
|---|---|
| Component | Content |
| PCR Buffer TB Green Premix Dimer Eraser^{*1} (manufactured by Takara Bio, Inc.) | 10.0µL |
| 10 µM Forward primer (manufactured by Riken Genesis Co., Ltd.) | 0.6µL |
| 10 µM Reverse primer (manufactured by Riken Genesis Co., Ltd.) | 0.6µL |
| ROX dye (manufactured by Takara Bio, Inc.) | 0.4µL |
| 10 µM BNA clamp (manufactured by Riken Genesis Co., Ltd.) | 0µL |
| Template DNA (50 ng/µL genomic DNA of HCC70 cells, or 50 ng/µL genomic DNA of MDU4475 cells) | 1.0µL |
| Polymer aqueous solution | 0µL |
| Nuclease-free water (manufactured by Thermo Fisher Scientific, Inc.) | 7.4µL |

| | |
|---|---|
| ^{*1} contains Taq polymerase (TaKaRa Ex Taq HS), dNTP Mixture, Mg²⁺, and a fluorescent dye (TB Green). | |

### 4-5. Real-Time PCR System and PCR Reaction

PCR reactions were carried out in the same manner as in Test 2, using the same system as that used in Test 2.

### 4-6. Calculation of ΔCt (WT) and ΔCt (Mutant) with or without Addition of Polymer, Calculation of ΔΔCt with or without Addition of Polymer, and Calculation of ΔΔΔCt

The same procedure as that used in Test 2 was carried out.

### 4-7. Results

The test results are summarized below.

**[Table 23]**

| | Polymer or monomer | ΔΔΔCt |
|---|---|---|
| Example 1 | Diallylmethylamine hydrochloride/sulfur dioxide copolymer | 7.76 |

As described above, the effect of selectively inhibiting amplification of the wild-type BRAF gene by BNA clamp was remarkably enhanced by the addition of the polymer of Example 1, similarly to the case of KRAS gene.

### 5. Adaptability to Mutant gene Other Than KRAS 2 (Sensitivity Enhancement of BNA Clamp PCR by Addition of Polymer)

### 5-1. Polymer Used

The polymer of Example 1 was dissolved in water, and a 0.1% by mass polymer solution was prepared, which was used for the preparation of a reaction mixture.

### 5-2. Kit Used

The same kit as that used in Test 4 was used.

### 5-3. Template DNA

The same template DNA as that used in Test 4 was used.

In this test, the template DNA of the wild-type gene and the template DNA of the mutant gene were mixed such that the % by mass of the template DNA of the mutant gene in the whole template DNAs was as shown in the following table while the whole amount of the template DNAs was adjusted to 50 ng in 2.0 µL. Each of the obtained template DNA mixtures, and the template DNA of the wild-type gene were used in PCR reactions.

**[Table 24]**

| Mutant ratio | Number of Mutant copies |
|---|---|
| 10% Mutant(5ng) | 1.65×10³ copies |
| 1% Mutant(0.5ng) | 1.65×10² copies |
| 0.1% Mutant(0.05ng) | 1.65×10 copies |
| 0.01% Mutant(0.005ng) | 1.65 copies |

### 5-4. Primers and BNA Clamp

The same primers and BNA clamp as those used in Test 4 were used.

### 5-5. BNA Clamp PCR Reaction Mixture

Reaction mixtures 1 to 4 with the compositions shown in the following table were prepared. The polymer concentration in the reaction mixture 1 was 0.01% by mass.

**[Table 25]**

| Reaction mixture 1 (polymer +, BNA clamp +) | |
|---|---|
| Component | Content |
| PCR Buffer TB Green Premix Dimer Eraser^{*1} (manufactured by Takara Bio, Inc.) | 10.0µL |
| 10 µM Forward primer (manufactured by Riken Genesis Co., Ltd.) | 0.6µL |
| 10 µM Reverse primer (manufactured by Riken Genesis Co., Ltd.) | 0.6µL |
| ROX dye (manufactured by Takara Bio, Inc.) | 0.4µL |
| 10 µM BNA clamp (manufactured by Riken Genesis Co., Ltd.) | 0.2µL |
| 50 ng of template DNA (WT^{*2} or mixture^{*4} of WT and Mutant^{*3}) | 2.0µL |
| 0.1% by mass polymer aqueous solution | 2.0µL |
| Nuclease-free water (manufactured by Thermo Fisher Scientific, Inc.) | 4.2µL |

| | |
|---|---|
| ^{*1} contains Taq polymerase (TaKaRa Ex Taq HS), dNTP Mixture, Mg²⁻, and a fluorescent dye (TB Green). ^{*2} Genomic DNA of HCC70 cells ^{*3} Genomic DNA of DU4475 cells ^{*4} contains 50 ng of the template DNA in 2.0 µL of whole mixture. | |

**[Table 26]**

| Reaction mixture 2 (polymer -, BNA clamp +) | |
|---|---|
| Component | Content |
| PCR Buffer TB Green Premix Dimer Eraser^{*1} (manufactured by Takara Bio, Inc.) | 10.0µL |
| 10 µM Forward primer (manufactured by Riken Genesis Co., Ltd.) | 0.6µL |
| 10 µM Reverse primer (manufactured by Riken Genesis Co., Ltd.) | 0.6µL |
| ROX dye (manufactured by Takara Bio, Inc.) | 0.4µL |
| 10 µM BNA clamp (manufactured by Riken Genesis Co., Ltd.) | 0.2µL |
| 50 ng of template DNA (WT^{*2} or mixture^{*4} of WT and Mutant^{*3}) | 2.0µL |
| Polymer aqueous solution | 0µL |
| Nuclease-free water (manufactured by Thermo Fisher Scientific, Inc.) | 6.2µL |

| | |
|---|---|
| ^{*1} contains Taq polymerase (TaKaRa Ex Taq HS), dNTP Mixture, Mg²⁻, and a fluorescent dye (TB Green). ^{*2} Genomic DNA of HCC70 cells ^{*3} Genomic DNA of DU4475 cells ^{*4} contains 50 ng of the template DNA in 2.0 µL of whole mixture. | |

### 5-6. Real-Time PCR System and PCR Reaction

PCR reactions were carried out in the same manner as in Test 2, using the same apparatus as that used in Test 2.

### 5-7. Determination of δCt (Mutant 10% to 0.01%) with or without addition of polymer and calculation of δδCt (Mutant 10% to 0.01%) with or without addition of polymer

The same procedure as that used in Test 3 was carried out.

### 5-8. Results

The test results are summarized in the following table and FIG. 3.

**[Table 27]**

| Mutant ratio | δCt in case of no addition of polymer | δCt in case of addition of polymer | δδCt |
|---|---|---|---|
| 10% Mutant | 10.76 | 14.87 | 4.11 |
| 1% Mutant | 6.81 | 11.33 | 4.52 |
| 0.1% Mutant | 4.60 | 7.74 | 3.14 |
| 0.01% Mutant | -3.75 | 2.96 | 6.71 |

As a result of this test, it became clear that in the case of no addition of polymer, when the Mutant ratio was 0.01%, δCt was less than 0, and the amplification curve of the mutant gene could not be distinguished from the amplification curve of the wild-type gene; however, in the case where the polymer of Example 1 was added, the amplification curve of the mutant gene could be distinguished from the amplification curve of the wild-type gene even at a Mutant ratio of 0.01%.

## Claims

1. A method for detecting mutation in a target base sequence of a nucleic acid in a sample relative to a standard base sequence, comprising the steps of:
subjecting the nucleic acid in the sample to a nucleic acid amplification reaction as a template with
a clamp nucleic acid having a base sequence complementary to the standard base sequence and containing at least one artificial nucleotide residue, and
a diallylamine/sulfur dioxide copolymer that comprises
a constituent unit (A) having a structure represented by general formula (I-a) or general formula (I-b), or a structure of an acid addition salt thereof: 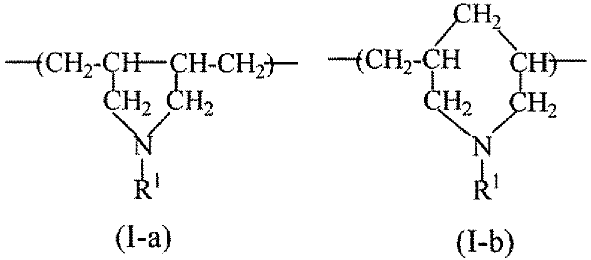
wherein R¹ represents an alkyl group having 1 to 10 carbon atoms, and
a constituent unit (B) having a structure represented by general formula (I-c): 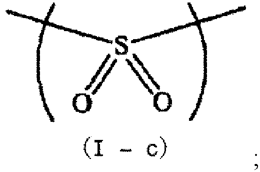 and
determining the presence of the mutation based on the total amount of nucleic acid amplification products obtained by the nucleic acid amplification reaction, the number of amplification cycles of the nucleic acid amplification reaction required for the total amount of the nucleic acid amplification products to reach a threshold value, the amount of nucleic acids with the mutation in the nucleic acid amplification products or the number of amplification cycles of the nucleic acid amplification reaction required for the amount of nucleic acids with the mutation in the nucleic acid amplification products to reach a threshold value.

2. The method according to claim 1, wherein the diallylamine/sulfur dioxide copolymer is a diallylmethylamine/sulfur dioxide copolymer, a diallylmethylamine acid addition salt/sulfur dioxide copolymer, a diallylethylamine/sulfur dioxide copolymer, or a diallylethylamine acid addition salt/sulfur dioxide copolymer, preferably a diallylmethylamine/sulfur dioxide copolymer or a diallylmethylamine acid addition salt/sulfur dioxide copolymer.

3. The method according to claim 1 or 2, wherein
(i) the artificial nucleotide is a BNA; or
(ii) the nucleic acid amplification is performed by real-time PCR; or
(iii) the nucleic acid in the sample is a genomic DNA; or
(iv) when performing the nucleic acid amplification reaction, a nucleic acid with the standard base sequence is further subjected to a nucleic acid amplification reaction as a template, wherein when the number of amplification cycles of the nucleic acid amplification reaction required for the total amount of the nucleic acid amplification products in the case of using the nucleic acid in the sample as a template to reach a threshold value is smaller than the number of amplification cycles of the nucleic acid amplification reaction required for the total amount of the nucleic acid amplification products in the case of using the nucleic acid with the standard base sequence as a template to reach a threshold value, it is determined that the mutation is present in the target base sequence of the nucleic acid in the sample.

4. A kit for:
(i) detecting mutation in a target base sequence of a nucleic acid in a sample relative to a standard base sequence, comprising a clamp nucleic acid having a base sequence complementary to the standard base sequence and containing at least one artificial nucleotide residue; or
(ii) inhibiting amplification of a nucleic acid having a target base sequence in a nucleic acid amplification reaction, comprising a clamp nucleic acid having a base sequence complementary to the target base sequence and containing at least one artificial nucleotide residue;
wherein the kit comprises
a diallylamine/sulfur dioxide copolymer that comprises
a constituent unit (A) having a structure represented by general formula (I-a) or general formula (I-b), or a structure of an acid addition salt thereof: 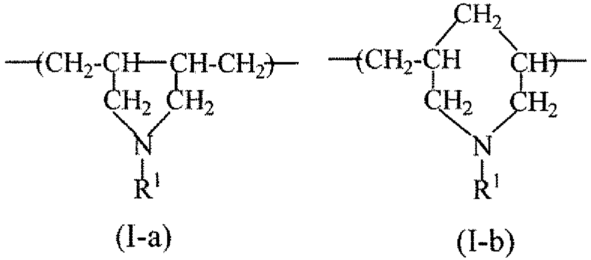
wherein R¹ represents an alkyl group having 1 to 10 carbon atoms, and
a constituent unit (B) having a structure represented by general formula (I-c):

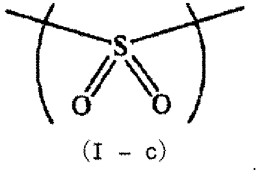

5. The kit according to claim 4, wherein the diallylamine/sulfur dioxide copolymer is a diallylmethylamine/sulfur dioxide copolymer, a diallylmethylamine acid addition salt/sulfur dioxide copolymer, a diallylethylamine/sulfur dioxide copolymer, or a diallylethylamine acid addition salt/sulfur dioxide copolymer, preferably a diallylmethylamine/sulfur dioxide copolymer or a diallylmethylamine acid addition salt/sulfur dioxide copolymer.

6. The kit according to claim 4 or 5, wherein the artificial nucleotide is a BNA.

7. A method for inhibiting amplification of a nucleic acid in a sample with a predetermined target base sequence in a nucleic acid amplification reaction, the method comprising
subjecting the nucleic acid to a nucleic acid amplification reaction as a template with
a clamp nucleic acid having a base sequence complementary to the target base sequence and containing at least one artificial nucleotide residue, and
a diallylamine/sulfur dioxide copolymer that comprises
a constituent unit (A) having a structure represented by general formula (I-a) or general formula (I-b), or a structure of an acid addition salt thereof: 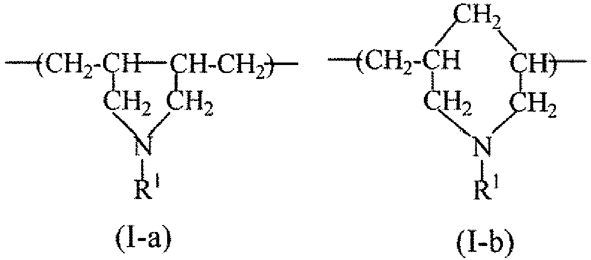
wherein R¹ represents an alkyl group having 1 to 10 carbon atoms, and
a constituent unit (B) having a structure represented by general formula (I-c):

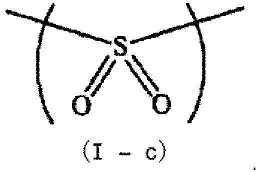

8. The method according to claim 7, wherein the diallylamine/sulfur dioxide copolymer is a diallylmethylamine/sulfur dioxide copolymer, a diallylmethylamine acid addition salt/sulfur dioxide copolymer, a diallylethylamine/sulfur dioxide copolymer, or a diallylethylamine acid addition salt/sulfur dioxide copolymer, preferably a diallylmethylamine/sulfur dioxide copolymer or a diallylmethylamine acid addition salt/sulfur dioxide copolymer.

9. The method according to claim 7 or 8, wherein the artificial nucleotide is a BNA.

10. Use of a nucleic acid amplification inhibition enhancing agent for enhancing a nucleic acid amplification inhibitory effect by a clamp nucleic acid, comprising:
a diallylamine/sulfur dioxide copolymer that comprises
a constituent unit (A) having a structure represented by general formula (I-a) or general formula (I-b), or a structure of an acid addition salt thereof: 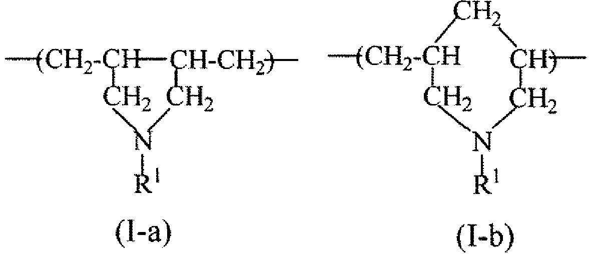
wherein R¹ represents an alkyl group having 1 to 10 carbon atoms, and
a constituent unit (B) having a structure represented by general formula (I-c):

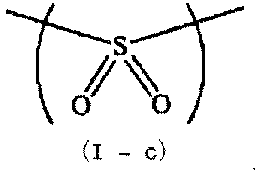

11. The use according to claim 10, wherein the diallylamine/sulfur dioxide copolymer is a diallylmethylamine/sulfur dioxide copolymer, a diallylmethylamine acid addition salt/sulfur dioxide copolymer, a diallylethylamine/sulfur dioxide copolymer, or a diallylethylamine acid addition salt/sulfur dioxide copolymer, preferably a diallylmethylamine/sulfur dioxide copolymer or a diallylmethylamine acid addition salt/sulfur dioxide copolymer.

## Patentansprüche

1. Verfahren zum Nachweisen einer Mutation in einer Zielbasensequenz einer Nukleinsäure in einer Probe relativ zu einer Standardbasensequenz, umfassend die Schritte von:
Unterziehen der Nukleinsäure in der Probe einer Nukleinsäure-Amplifikationsreaktion als Matrize mit einer Nukleinsäure-Klammer ("clamp nucleic acid"), die eine zur Standardbasensequenz komplementäre Basensequenz aufweist und mindestens einen künstlichen Nukleotidrest enthält, und
einem Diallylamin/Schwefeldioxid-Copolymer, das umfasst eine Struktureinheit ("constituent unit") (A) mit einer Struktur, die durch die allgemeine Formel (I-a) oder die allgemeine Formel (I-b) dargestellt wird, oder eine Struktur eines Säureadditionssalzes davon: 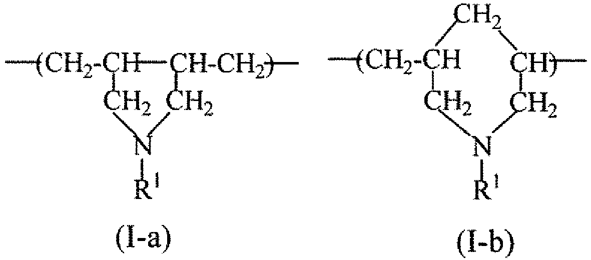
wobei R¹ eine Alkylgruppe mit 1 bis 10 Kohlenstoffatomen darstellt, und
eine Struktureinheit (B) mit einer Struktur, die durch die allgemeine Formel (I-c) dargestellt wird: 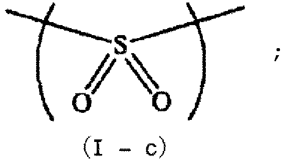 und
Bestimmen des Vorhandenseins der Mutation basierend auf der Gesamtmenge der durch die Nukleinsäure-Amplifikationsreaktion erhaltenen Nukleinsäure-Amplifikationsprodukte, der Anzahl der Amplifikationszyklen der Nukleinsäure-Amplifikationsreaktion, die erforderlich ist, damit die Gesamtmenge der Nukleinsäure-Amplifikationsprodukte einen Schwellenwert erreicht, der Menge an Nukleinsäuren mit der Mutation in den Nukleinsäure-Amplifikationsprodukten oder der Anzahl der Amplifikationszyklen der Nukleinsäure-Amplifikationsreaktion, die erforderlich ist, damit die Menge an Nukleinsäuren mit der Mutation in den Nukleinsäure-Amplifikationsprodukten einen Schwellenwert erreicht.

2. Verfahren gemäß Anspruch 1, wobei das Diallylamin/Schwefeldioxid-Copolymer ein Diallylmethylamin/Schwefeldioxid-Copolymer, ein Diallylmethylamin-Säureadditionssalz/Schwefeldioxid-Copolymer, ein Diallylethylamin/Schwefeldioxid-Copolymer oder ein Diallylethylamin-Säureadditionssalz/Schwefeldioxid-Copolymer ist, vorzugsweise ein Diallylmethylamin/Schwefeldioxid-Copolymer oder ein Diallylmethylamin-Säureadditionssalz/Schwefeldioxid-Copolymer.

3. Verfahren gemäß Anspruch 1 oder 2, wobei
(i) das künstliche Nukleotid ein BNA ist; oder
(ii) die Nukleinsäure-Amplifikation mittels Echtzeit-PCR ("real-time PCR") durchgeführt wird; oder
(iii)die Nukleinsäure in der Probe eine genomische DNA ist; oder
(iv) bei der Durchführung der Nukleinsäure-Amplifikationsreaktion eine Nukleinsäure mit der Standardbasensequenz als Matrize einer weiteren Nukleinsäure-Amplifikationsreaktion unterzogen wird, wobei, wenn die Anzahl der Amplifikationszyklen der Nukleinsäure-Amplifikationsreaktion, die erforderlich ist, damit die Gesamtmenge der Nukleinsäure-Amplifikationsprodukte im Falle der Verwendung der Nukleinsäure in der Probe als Matrize einen Schwellenwert erreicht, kleiner ist als die Anzahl der Amplifikationszyklen der Nukleinsäure-Amplifikationsreaktion, die erforderlich ist, damit die Gesamtmenge der Nukleinsäure-Amplifikationsprodukte im Falle der Verwendung der Nukleinsäure mit der Standardbasensequenz als Matrize einen Schwellenwert erreicht, festgestellt wird, dass die Mutation in der Zielbasensequenz der Nukleinsäure in der Probe vorliegt.

4. Kit zum:
(i) Nachweisen einer Mutation in einer Zielbasensequenz einer Nukleinsäure in einer Probe realtiv zu einer Standardbasensequenz, umfassend eine Nukleinsäure-Klammer, die eine zur Standardbasensequenz komplementäre Basensequenz aufweist und mindestens einen künstlichen Nukleotidrest enthält; oder
(ii) Hemmen der Amplifikation einer Nukleinsäure mit einer Zielbasensequenz in einer Nukleinsäure-Amplifikationsreaktion, umfassend eine Nukleinsäure-Klammer, die eine zur Zielbasensequenz komplementäre Basensequenz aufweist und mindestens einen künstlichen Nukleotidrest enthält;
wobei das Kit umfasst
einem Diallylamin/Schwefeldioxid-Copolymer, das umfasst: eine Struktureinheit ("constituent unit") (A) mit einer Struktur, die durch die allgemeine Formel (I-a) oder die allgemeine Formel (I-b) dargestellt wird, oder eine Struktur eines Säureadditionssalzes davon: 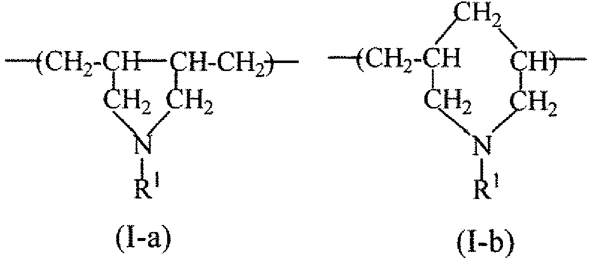
wobei R¹ eine Alkylgruppe mit 1 bis 10 Kohlenstoffatomen darstellt, und
eine Struktureinheit (B) mit einer Struktur, die durch die allgemeine Formel (I-c) dargestellt wird:

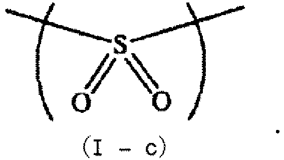

5. Kit gemäß Anspruch 4, wobei das Diallylamin/Schwefeldioxid-Copolymer ein Diallylmethylamin/Schwefeldioxid-Copolymer, ein Diallylmethylamin-Säureadditionssalz/Schwefeldioxid-Copolymer, ein Diallylethylamin/Schwefeldioxid-Copolymer oder ein Diallylethylamin-Säureadditionssalz/Schwefeldioxid-Copolymer ist, vorzugsweise ein Diallylmethylamin/Schwefeldioxid-Copolymer oder ein Diallylmethylamin-Säureadditionssalz/Schwefeldioxid-Copolymer.

6. Kit gemäß Anspruch 4 oder 5, wobei das künstliche Nukleotid ein BNA ist.

7. Verfahren zum Hemmen der Amplifikation einer Nukleinsäure in einer Probe mit einer vorbestimmten Zielbasensequenz in einer Nukleinsäure-Amplifikationsreaktion, das Verfahren umfassend Unterziehen der Nukleinsäure einer Nukleinsäure-Amplifikationsreaktion als Matrize mit einer Nukleinsäure-Klammer, die eine zur Zielbasensequenz komplementäre Basensequenz aufweist und mindestens einen künstlichen Nukleotidrest enthält, und einem Diallylamin/Schwefeldioxid-Copolymer, das umfasst eine Struktureinheit ("constituent unit") (A) mit einer Struktur, die durch die allgemeine Formel (I-a) oder die allgemeine Formel (I-b) dargestellt wird, oder eine Struktur eines Säureadditionssalzes davon: 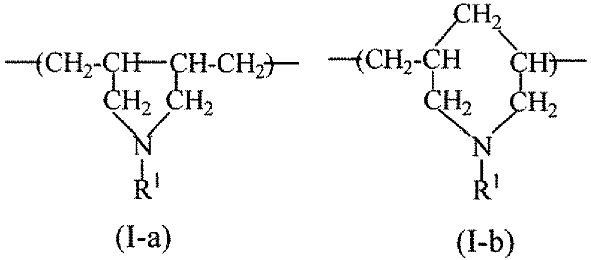
wobei R¹ eine Alkylgruppe mit 1 bis 10 Kohlenstoffatomen darstellt, und
eine Struktureinheit (B) mit einer Struktur, die durch die allgemeine Formel (I-c) dargestellt wird:

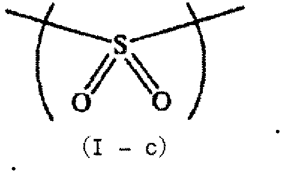

8. Verfahren gemäß Anspruch 7, wobei das Diallylamin/Schwefeldioxid-Copolymer ein Diallylmethylamin/Schwefeldioxid-Copolymer, ein Diallylmethylamin-Säureadditionssalz/Schwefeldioxid-Copolymer, ein Diallylethylamin/Schwefeldioxid-Copolymer oder ein Diallylethylamin-Säureadditionssalz/Schwefeldioxid-Copolymer ist, vorzugsweise ein Diallylmethylamin/Schwefeldioxid-Copolymer oder ein Diallylmethylamin-Säureadditionssalz/Schwefeldioxid-Copolymer.

9. Verfahren gemäß Anspruch 7 oder 8, wobei das künstliche Nukleotid ein BNA ist.

10. Verwendung eines Agens zur Verstärkung der Hemmung der Nukleinsäure-Amplifikation zur Verstärkung der hemmenden Wirkung einer Nukleinsäure-Klammer auf die Nukleinsäure-Amplifikation, umfassend:
einem Diallylamin/Schwefeldioxid-Copolymer, das umfasst eine Struktureinheit ("constituent unit") (A) mit einer Struktur, die durch die allgemeine Formel (I-a) oder die allgemeine Formel (I-b) dargestellt wird, oder eine Struktur eines Säureadditionssalzes davon: 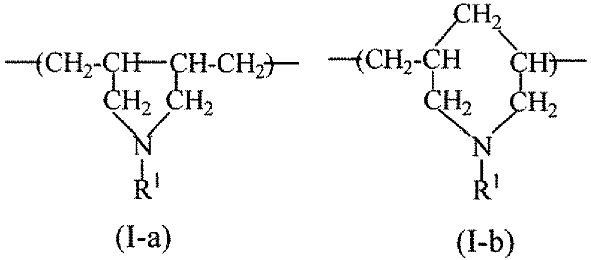
wobei R¹ eine Alkylgruppe mit 1 bis 10 Kohlenstoffatomen darstellt, und
eine Struktureinheit (B) mit einer Struktur, die durch die allgemeine Formel (I-c) dargestellt wird:

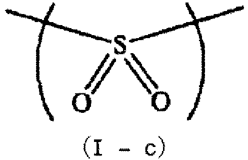

11. Verwendung gemäß Anspruch 10, wobei das Diallylamin/Schwefeldioxid-Copolymer ein Diallylmethylamin/Schwefeldioxid-Copolymer, ein Säureadditionssalz von Diallylmethylamin/Schwefeldioxid-Copolymer, ein Diallylethylamin/Schwefeldioxid-Copolymer oder ein Diallylethylamin-Säureadditionssalz/Schwefeldioxid-Copolymer ist, vorzugsweise ein Diallylmethylamin/Schwefeldioxid-Copolymer oder ein Diallylmethylamin-Säureadditionssalz/Schwefeldioxid-Copolymer.

## Revendications

1. Procédé de détection d'une mutation dans une séquence de bases cible d'un acide nucléique dans un échantillon par rapport à une séquence de bases standard, comprenant les étapes :
de soumission de l'acide nucléique dans l'échantillon à une réaction d'amplification d'acide nucléique en tant que matrice avec
un acide nucléique de blocage présentant une séquence de bases complémentaire de la séquence de bases standard et contenant au moins un résidu nucléotidique artificiel, et
un copolymère de diallylamine/de dioxyde de soufre qui comprend
une unité constitutive (A) présentant une structure représentée par la formule générale (I-a) ou la formule générale (I-b), ou une structure d'un sel d'addition d'acide de celui-ci : 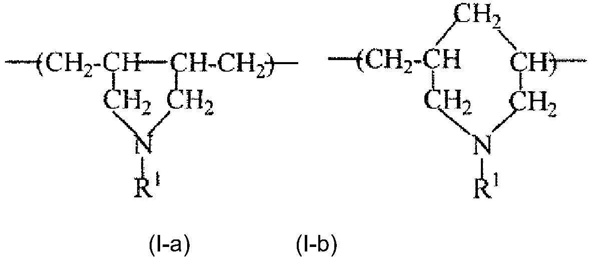
dans lequel R¹ représente un groupe alkyle présentant 1 à 10 atomes de carbone, et
une unité constitutive (B) présentant une structure représentée par la formule générale (I-c) : 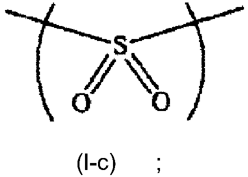 et
de détermination de la présence de la mutation sur la base de la quantité totale de produits d'amplification d'acide nucléique obtenus par la réaction d'amplification d'acide nucléique, du nombre de cycles d'amplification de la réaction d'amplification d'acide nucléique nécessaires pour que la quantité totale des produits d'amplification d'acide nucléique atteigne une valeur seuil, de la quantité d'acides nucléiques avec la mutation dans les produits d'amplification d'acide nucléique ou du nombre de cycles d'amplification de la réaction d'amplification d'acide nucléique nécessaires pour que la quantité d'acides nucléiques avec la mutation dans les produits d'amplification d'acide nucléique atteigne une valeur seuil.

2. Procédé selon la revendication 1, dans lequel le copolymère de diallylamine/de dioxyde de soufre est un copolymère de diallylméthylamine/de dioxyde de soufre, un copolymère de sel d'addition d'acide de diallylméthylamine/de dioxyde de soufre, un copolymère de diallyléthylamine/de dioxyde de soufre, ou un copolymère de sel d'addition d'acide de diallyléthylamine/de dioxyde de soufre, de préférence un copolymère de diallylméthylamine/de dioxyde de soufre ou un copolymère de sel d'addition d'acide de diallylméthylamine/de dioxyde de soufre.

3. Procédé selon la revendication 1 ou la revendication 2, dans lequel
(i) le nucléotide artificiel est un BNA ; ou
(ii) l'amplification d'acide nucléique est effectuée par PCR en temps réel ; ou
(iii) l'acide nucléique dans l'échantillon est un ADN génomique ; ou
(iv) lors de la réalisation de la réaction d'amplification d'acide nucléique, un acide nucléique présentant la séquence de bases standard est en outre soumis à une réaction d'amplification d'acide nucléique en tant que matrice, dans lequel, lorsque le nombre de cycles d'amplification de la réaction d'amplification d'acide nucléique nécessaires pour que la quantité totale des produits d'amplification d'acide nucléique dans le cas de l'utilisation de l'acide nucléique dans l'échantillon en tant que matrice atteigne une valeur seuil est inférieur au nombre de cycles d'amplification de la réaction d'amplification d'acide nucléique nécessaires pour que la quantité totale des produits d'amplification d'acide nucléique dans le cas de l'utilisation de l'acide nucléique avec la séquence de bases standard en tant que matrice atteigne une valeur seuil, il est déterminé que la mutation est présente dans la séquence de bases cible de l'acide nucléique dans l'échantillon.

4. Kit pour :
(i) la détection d'une mutation dans une séquence de bases cible d'un acide nucléique dans un échantillon par rapport à une séquence de bases standard, comprenant un acide nucléique de blocage présentant une séquence de bases complémentaire de la séquence de bases standard et contenant au moins un résidu nucléotidique artificiel ; ou
(ii) l'inhibition de l'amplification d'un acide nucléique présentant une séquence de bases cible dans une réaction d'amplification d'acide nucléique, comprenant un acide nucléique de blocage présentant une séquence de bases complémentaire de la séquence de bases cible et contenant au moins un résidu nucléotidique artificiel ;
dans lequel le kit comprend
un copolymère de diallylamine/de dioxyde de soufre qui comprend
une unité constitutive (A) présentant une structure représentée par la formule générale (I-a) ou la formule générale (I-b), ou une structure d'un sel d'addition d'acide de celui-ci : 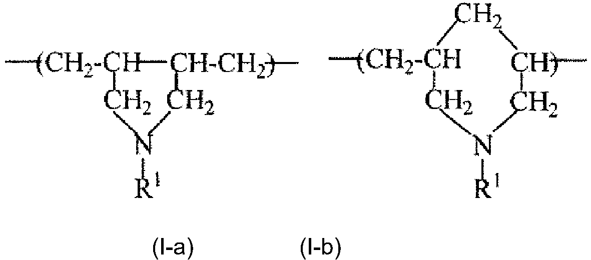
dans lequel R¹ représente un groupe alkyle présentant 1 à 10 atomes de carbone, et
une unité constitutive (B) présentant une structure représentée par la formule générale (I-c) :

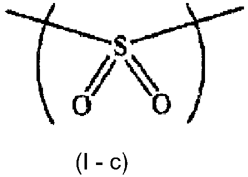

5. Kit selon la revendication 4, dans lequel le copolymère de diallylamine/de dioxyde de soufre est un copolymère de diallylméthylamine/de dioxyde de soufre, un copolymère de sel d'addition d'acide de diallylméthylamine/de dioxyde de soufre, un copolymère de diallyléthylamine/de dioxyde de soufre, ou un copolymère de sel d'addition d'acide de diallyléthylamine/de dioxyde de soufre, de préférence un copolymère de diallylméthylamine/de dioxyde de soufre ou un copolymère de sel d'addition d'acide de diallylméthylamine/de dioxyde de soufre.

6. Kit selon la revendication 4 ou la revendication 5, dans lequel le nucléotide artificiel est un BNA.

7. Procédé d'inhibition de l'amplification d'un acide nucléique dans un échantillon avec une séquence de bases cible prédéterminée dans une réaction d'amplification d'acide nucléique, le procédé comprenant
la soumission de l'acide nucléique à une réaction d'amplification d'acide nucléique en tant que matrice avec un acide nucléique de blocage présentant une séquence de bases complémentaire de la séquence de bases cible et contenant au moins un résidu nucléotidique artificiel, et
un copolymère de diallylamine/de dioxyde de soufre qui comprend
une unité constitutive (A) présentant une structure représentée par la formule générale (I-a) ou la formule générale (I-b), ou une structure d'un sel d'addition d'acide de celui-ci : 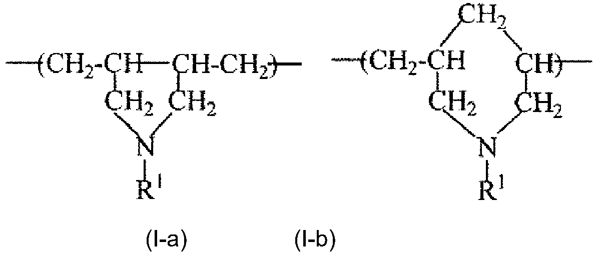
dans lequel R¹ représente un groupe alkyle présentant 1 à 10 atomes de carbone, et
une unité constitutive (B) présentant une structure représentée par la formule générale (I-c) :

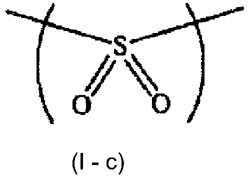

8. Procédé selon la revendication 7, dans lequel le copolymère de diallylamine/de dioxyde de soufre est un copolymère de diallylméthylamine/de dioxyde de soufre, un copolymère de sel d'addition d'acide de diallylméthylamine/de dioxyde de soufre, un copolymère de diallyléthylamine/de dioxyde de soufre, ou un copolymère de sel d'addition d'acide de diallyléthylamine/de dioxyde de soufre, de préférence un copolymère de diallylméthylamine/de dioxyde de soufre ou un copolymère de sel d'addition d'acide de diallylméthylamine/de dioxyde de soufre.

9. Procédé selon la revendication 7 ou la revendication 8, dans lequel le nucléotide artificiel est un BNA.

10. Utilisation d'un agent amplificateur de l'inhibition de l'amplification d'acide nucléique pour amplifier un effet inhibiteur de l'amplification d'acide nucléique par un acide nucléique de blocage, comprenant :
un copolymère de diallylamine/de dioxyde de soufre qui comprend
une unité constitutive (A) présentant une structure représentée par la formule générale (I-a) ou la formule générale (I-b), ou une structure d'un sel d'addition d'acide de celui-ci : 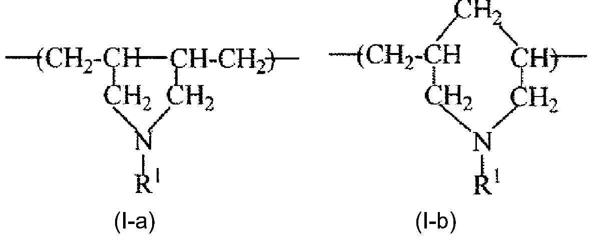
dans lequel R¹ représente un groupe alkyle présentant 1 à 10 atomes de carbone, et
une unité constitutive (B) présentant une structure représentée par la formule générale (I-c) :

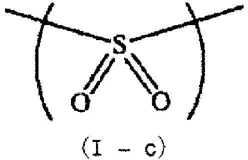

11. Utilisation selon la revendication 10, dans laquelle le copolymère de diallylamine/de dioxyde de soufre est un copolymère de diallylméthylamine/de dioxyde de soufre, un copolymère de sel d'addition d'acide de diallylméthylamine/de dioxyde de soufre, un copolymère de diallyléthylamine/de dioxyde de soufre, ou un copolymère de sel d'addition d'acide de diallyléthylamine/de dioxyde de soufre, de préférence un copolymère de diallylméthylamine/de dioxyde de soufre ou un copolymère de sel d'addition d'acide de diallylméthylamine/de dioxyde de soufre.
